# EUROPEAN PATENT SPECIFICATION

(11) **EP 3 874 058 B1**
(45) Date of publication and mention of the grant of the patent: **31.12.2025**
(21) Application number: 19899577.1
(22) Date of filing: 01.11.2019
(51) Int. Cl.: C12Q 1/6813, C40B 20/04, C40B 70/00, C40B 40/06

(54) **NUCLEIC ACID-BASED BARCODING**
NUKLEINSÄUREBASIERTE BARCODIERUNG
CODAGE À BARRES RÉALISÉ AVEC DE L'ACIDE NUCLÉIQUE

(30) Priority: 01.11.2018 US 201862754450 P
(43) Date of publication of application: 08.09.2021
(73) Proprietor: President And Fellows Of Harvard College, Cambridge, Massachusetts 02138 (US)
(72) Inventor: LIU, Ninning, Boston, Massachusetts 02215 (US); YIN, Peng, Brookline, Massachusetts 02445 (US); SAKA, Sinem K., Allston, Massachusetts 02134 (US); KISHI, Jocelyn Yoshiko, Boston, Massachusetts 02215 (US)
(74) Representative: Brand Murray Fuller LLP
(86) International application number: PCT/US2019/059484
(87) International publication number: WO 2020/131233

(56) References cited:
- US-A1- 2016 312 272
- US-A1- 2017 267 997
- US-A1- 2017 267 997
- YOSHIMURA YOSHINAGA ET AL: "Ultrafast Reversible Photo-Cross-Linking Reaction: Toward in Situ DNA Manipulation", vol. 10, no. 15, 7 August 2008 (2008-08-07), US, pages 3227 - 3230, XP055809920, ISSN: 1523-7060, Retrieved from the Internet <URL:https://pubs.acs.org/doi/pdf/10.1021/ol801112j> DOI: 10.1021/ol801112j
- BYSTRYKH: "Generalized DNA barcode design based on Hamming codes", PLOS ONE, vol. 7, no. 5, 17 May 2012 (2012-05-17), pages e36852 1 - 8, XP055182966, DOI: 10.1371/journal.pone.0036852

## Description

### CROSS-REFERENCE TO RELATED APPLICATIONS

This application claims benefit under 35 U.S.C. § 119(e) of U.S. Provisional Application No. 62/754,450 filed November 1, 2018.

### GOVERNMENT SUPPORT

This invention was made with government support under Contract No. N00014-16-1-2410 awarded by Office of Naval Research and Grant No. CCF-1317291, awarded by National Science Foundation. The government has certain rights in the invention.

### TECHNICAL FIELD

The present disclosure relates to compositions and methods of nucleic acid barcoding.

### BACKGROUND

Nucleic acids have been proposed as a promising substrate for long-term data storage, given the incredible density of information they can hold and their stability over long periods of time [1]. Herein, a method is presented for fast, light-directed concatenation of nucleic acid data barcodes. This method of light-directed nucleic acid data writing is compatible with, and can be performed in aqueous media (e.g., water). Nucleic acid data barcodes can then be printed on various forms of aqueous media, including compressible hydrogels, that can be patterned with nucleic acid-encoded data to reduce the physical space the information takes up, thus increasing the information density of the material. Shrunken, and often dehydrated, hydrogels can be stored for long periods of time before being rehydrated and expanded to read the data stored in them. This cost-effective method for physical data compression has the potential to greatly exceed the information density of conventional data storage solutions (*e.g*. blu-ray) and represents a new type of nucleic acid-encoded data storage.

Nucleic acids, *e.g.,* DNA, have some of the highest volumetric data density in terms of bytes/gram, reaching a theoretical maximum of ~455 exabytes/gram [2]. However, both the speed and cost of nucleic acid synthesis are slow and expensive, which has historically prohibited nucleic acids from use as a viable data storage method. To mitigate these issues, herein are described nucleic acid data storage methodologies that aim to synthesize nucleic acid data barcode concatemers using only nucleic acids, a photo-reactive element, and light. These methods are enzyme-free and compatible with aqueous media, thus enabling nucleic acid barcode synthesis without requiring specialized and toxic chemicals that have been common in conventional chemical synthesis of nucleic acids, or the use of expensive enzymes. As a general purpose digital data storage platform, nucleic acid data storage would be relevant towards any number of industries. Of particular interest, these strategies would be useful in archival storage, where the low maintenance cost and long-term stability of nucleic acids would be desirable

US 2017/267997 A1 discloses a method for combinatorial construction of genetic elements.

### SUMMARY

Some aspects of the present disclosure provide a method of writing data, comprising: photocrosslinking a first nucleic acid comprising a barcode flanked by a pair of hybridization domains to a second nucleic acid comprising a barcode domain (*e.g*., DNA data domain) flanked by a pair of hybridization domains; optionally further comprising photocrosslinking to the first or second nucleic acid at least one additional nucleic acid that comprises a barcode flanked by a pair of hybridization domains, wherein one of the hybridization domains of each pair comprises a photoreactive element, and wherein each barcode is assigned an independent bit value; and producing a concatemer of barcodes.

In another aspect, provided herein is a method comprising writing data on or within a substrate with nucleic acids that encode data.

In another aspect, provided herein is a method comprising reading nucleic acids that encode data which are written, optionally patterned, on a substrate or compressible hydrogel

In another aspect, provided herein is a method comprising compressing a substrate or a compressible hydrogel that is written, optionally patterned, with nucleic acids that encode data.

In another aspect, provided herein is a method comprising reading nucleic acids that encode data which are written, optionally patterned, on a substrate.

In yet another aspect, provided herein is barcoding method comprising:
a. attaching a first nucleic acid comprising a barcode and a hybridization domain to a substrate;
b. photocrosslinking the first nucleic acid to a second nucleic acid comprising a barcode domain flanked by a pair of hybridization domains;

wherein one of the hybridization domains of each pair comprises a photoreactive element,
wherein each barcode is assigned an independent bit value;
and wherein the photocrosslinking produces a concatemer of barcodes.

In another aspect, provided herein is a barcoded substrate made by the methods described herein.

In another aspect, provided herein is a substrate patterned with nucleic acids that encode data.

Additional aspects provide a compressible hydrogel patterned with nucleic acids that encode data.

In another aspect, provided herein is a barcode composition comprising:
a. a first nucleic acid comprising a barcode domain flanked by a pair of hybridization domains;
b. a second nucleic acid comprising a barcode domain flanked by a pair of hybridization domains;
c. a substrate;
d. optionally, an agent, wherein the agent permits attachment of the first nucleic acid to the substrate;
e. optionally, at least one additional nucleic acid that comprises a barcode domain flanked by a pair of hybridization domains;

wherein each barcode domain is assigned an independent bit value,
wherein at least one of the hybridization domains of each nucleic acid comprises a photoreactive element,
and wherein at least one nucleic acid is immobilized to the substrate at a predetermined location.

In some embodiments of any of the aspects, the barcode is a DNA data barcode.

In some embodiments of any of the aspects, the photoreactive element is a photoreactive nucleotide.

In some embodiments of any of the aspects, the photoreactive nucleotide is a CNVK or CNVD crosslinking base.

In some embodiments of any of the aspects, the photocrosslinking is performed using a 350-400 nm, optionally a 365 nm, wavelength of light.

In some embodiments of any of the aspects, the photoreactive element is psoralen.

In some embodiments of any of the aspects, a DNA data barcode of the first and/or second nucleic acid is selected from a barcode library having a minimum Hamming distance of 4.

In some embodiments of any of the aspects, the photocrosslinking is performed in aqueous solution.

In some embodiments of any of the aspects, the method is enzyme-free.

In some embodiments of any of the aspects, each nucleic acid is irreversibly covalently linked to at least one other of the nucleic acid through a single photoreactive element of a hybridization domain.

In some embodiments of any of the aspects, each barcode has a length of at least 5 nucleotides, optionally 5-10 nucleotides.

In some embodiments of any of the aspects, the first and/or second nucleic acid is attached to a substrate.

In some embodiments of any of the aspects, the methods further comprise producing a concatemer of barcodes according to the method provided herein, wherein the first nucleic acid is linked to a docking strand on the substrate.

In some embodiments of any of the aspects, the concatemer of barcodes encode special information and/or spatial information. In some embodiments of any of the aspects, the special information is selected from the group consisting of: text, images, coordinates, graphics, movies, sequencing data, QR codes, binary codes, and health records.

In some embodiments of any of the aspects, the substrate is selected from the group consisting of: glass, transparent polymers, polystyrene, hydrogels, metal, ceramic, paper, agarose, gelatin, alginate, dextran, iron oxide, stainless steel, gold, copper, silver chloride, polycarbonate, polydimethylsiloxane, polyethylene, acrylonitrile butadiene styrene, cyclo-olefin polymers, cyclo-olefin copolymers, streptavidin, resin, and a biological material. In some embodiments of any of the aspects, the biological material is selected from the group consisting of: a tissue, a cell, an organoid, an engineered tissue; and an extracellular matrix. In some embodiments of any of the aspects, the substrate is selected from glass, transparent polymers, polystyrene, and hydrogels. In some embodiments of any of the aspects, the substrate is selected from compressible hydrogels.

In some embodiments of any of the aspects, the substrate or compressible hydrogel comprises a water-soluble polymer or a natural polymer. In some embodiments of any of the aspects, the water-soluble polymer is selected from poly(acrylic acid), poly(vinyl alcohol), poly(vinylpyrrolidone), poly(ethylene glycol), polyacrylamide, and polysaccharides. In some embodiments of any of the aspects, the natural polymer is selected from gelatin, agar, and collagen.

In some embodiments of any of the aspects, the writing of data on or within the substrate comprises linking or embedding docking strands to the substrate, optionally wherein the docking strands are linked or embedded uniformly or randomly to the substrate, and optionally wherein the docking strands comprise a modification that enables the docking strand to covalently link to the substrate, optionally an acrydite modification.

In some embodiments of any of the aspects, the methods further comprise compressing and optionally desiccating the substrate or hydrogel. In some embodiments of any of the aspects, the compressible substrate or hydrogel is chemically compressed, optionally by adding a non-aqueous solvent to the substrate or hydrogel or by increasing the total ionic concentration surrounding the substrate or hydrogel, or wherein the compressible substrate or hydrogel is physically compressed. In some embodiments of any of the aspects, the substrate or hydrogel is compressed in size by at least one-thousand-fold, by at least one-hundred-fold, or by at least ten-fold. In some embodiments of any of the aspects, the methods further comprise storing the substrate or hydrogel.

In some embodiments of any of the aspects, the substrate comprises at least 1 petabyte of data per cubic centimeter. In some embodiments of any of the aspects, the data is written at a 0.5 - 5 micron, optionally a 1 micron, resolution. In some embodiments of any of the aspects, the data is a data pattern, optionally wherein the nucleic acids that encode data are patterned on the substrate using a Digital Micromirror Device.

In some embodiments of any of the aspects, the first and/or second nucleic acid is attached to the substrate in a predetermined pattern. In some embodiments of any of the aspects, the nucleic acids are embedded on or within the substrate in a predetermined pattern. In some embodiments of any of the aspects, the predetermined pattern is a geometric shape, a square, a circle, or triangle. In some embodiments of any of the aspects, the predetermined pattern comprises repeating elements. In some embodiments of any of the aspects, the predetermined pattern is asymmetrical or symmetrical. In some embodiments of any of the aspects, the predetermined pattern comprises spatial information and/or special information. In some embodiments of any of the aspects, the first nucleic acid is attached at a 0.25 - 100 micron, optionally a 1 micron, resolution.

In some embodiments of any of the aspects, the encoded data is read using light microscopy, electron microscopy, atomic force microscopy, the unaided eye, or nucleic acid sequencing technology, optionally subsequent to cleavage of the nucleic acids from the substrate.

### BRIEF DESCRIPTION OF THE DRAWINGS

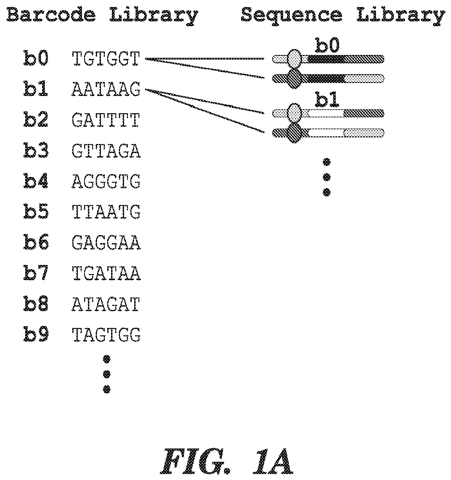

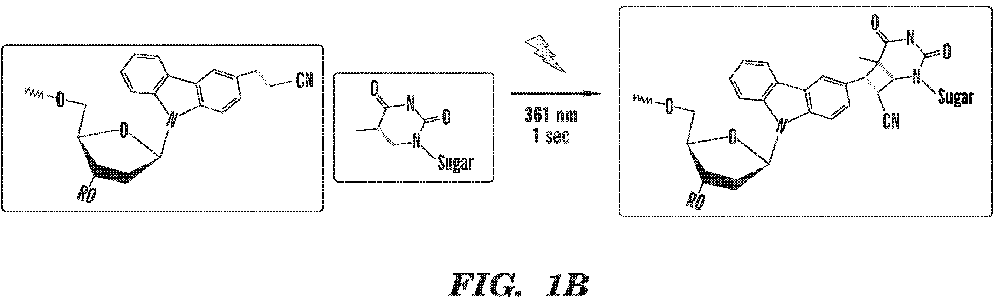

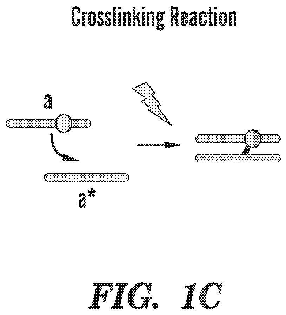

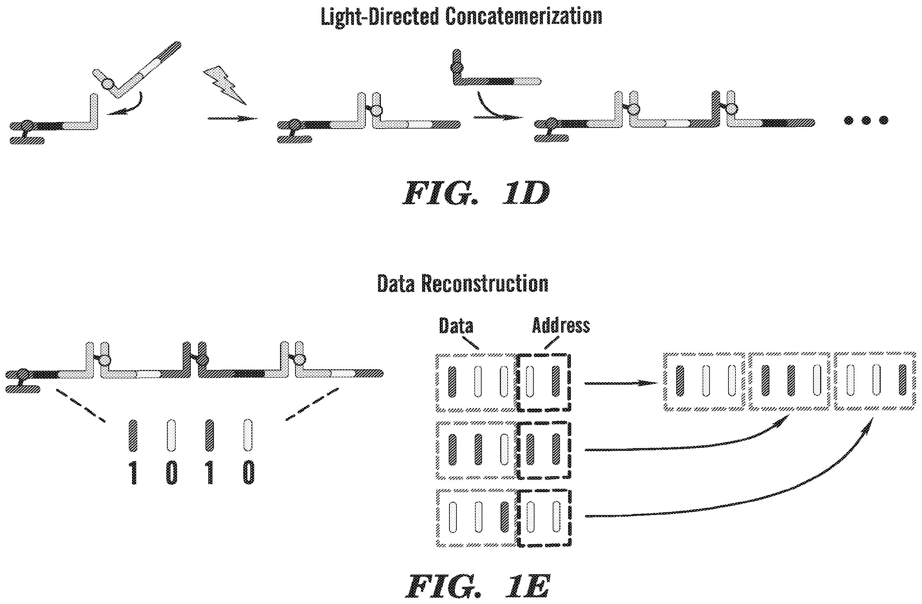

**FIGS. 1A-1E** provide an overview of light-directed data writing with DNA. **FIG. 1A** provide exemplary barcode library and nucleic acid design. Several example barcode sequences are shown. **FIG. 1B** shows a light-directed reaction between a CNVK base modification (left box) and a depicted thymine base (middle box) to produce a crosslinked nucleic acid (right box). **FIG. 1C** shows a cartoon of a CNVK crosslinking reaction. A DNA strand 'a' comprising a CNVK modification (filled circle) can form an interstrand crosslink with a complementary oligo 'a*'. **FIG. 1D** shows a schematic of light-directed concatemerization. **FIG. 1E** shows a schematic for data reconstruction of bit order of concatenated DNA barcodes.

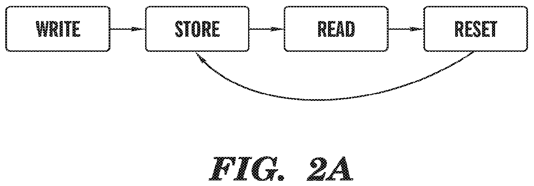

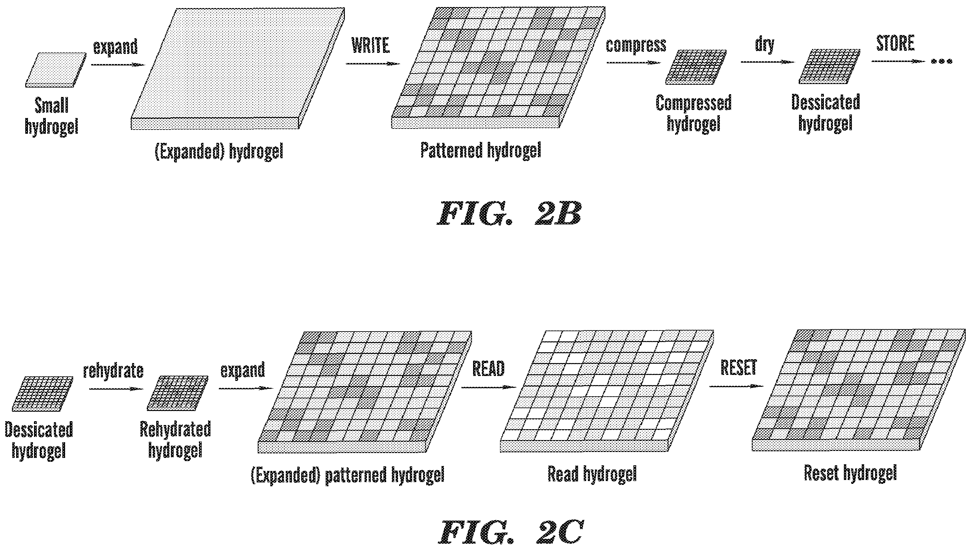

**FIGS. 2A-2C** depict an exemplary method for storing nucleic acid-encoded data on a compressible hydrogel. **FIG. 2A** shows that in a typical scenario, data is first written onto the hydrogel substrate (WRITE) before being stored (STORE). At the point the data needs to be reviewed, it is read (READ) and depending on the patterning method can additionally require a resetting (RESET) step prior to storage again. **FIG. 2B** illustrates how a small hydrogel can be expanded before the data (pattern) is applied (WRITE). After the data has been written, the gel is re-compressed and then stored in a desiccated state (STORE). **FIG. 2C** demonstrates that, in order to read the data pattern, desiccated hydrogels must typically be re-hydrated and expanded before being read. After reading of the information, the hydrogel can again be re-compressed and desiccated for further storage.

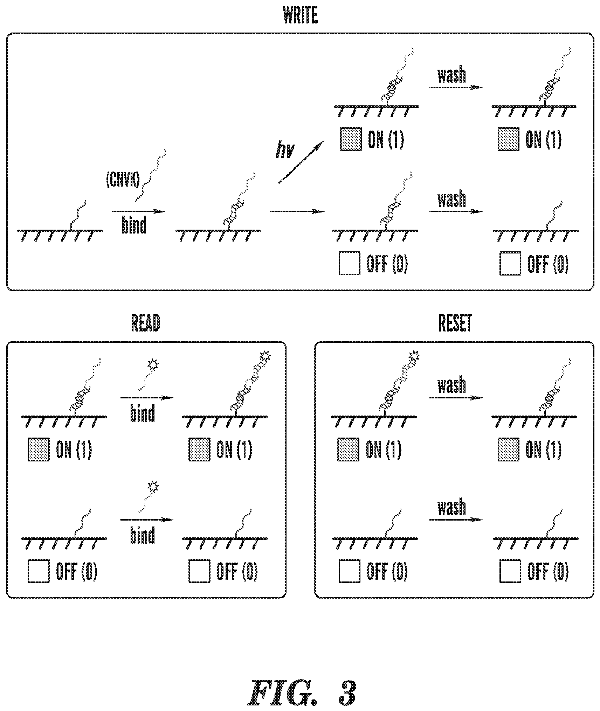

**FIG. 3** depicts exemplary light-directed WRITE, READ, and RESET operations using CNVK-modified strands. (WRITE) A crosslinking strand (CNVK) containing a CNVK modification within its complementary domain binds to a docking strand that is embedded in the hydrogel. Then, if and when the complex is exposed to UV light at 365 nm, a covalent interstrand crosslink forms between the strands. These cross-linked strands are stable enough to survive wash conditions (*e.g*. low salt, high temperature, formamide) and considered to be in the ON state, corresponding to a bit value of 1. Complexes not exposed to UV light do not become crosslinked and thus dissociate during the wash step. These docking strands are considered to be in the OFF state, corresponding to a bit value of 0. (READ) A crosslinked strand in the ON state can bind to a fluorescently labeled nucleic acid that is capable of hybridizing to the crosslinked nucleic acid, to allow for the strand to be read using a fluorescent imager. (RESET) A crosslinked strand in the ON state may be washed to remove the hybridized nucleic acid.

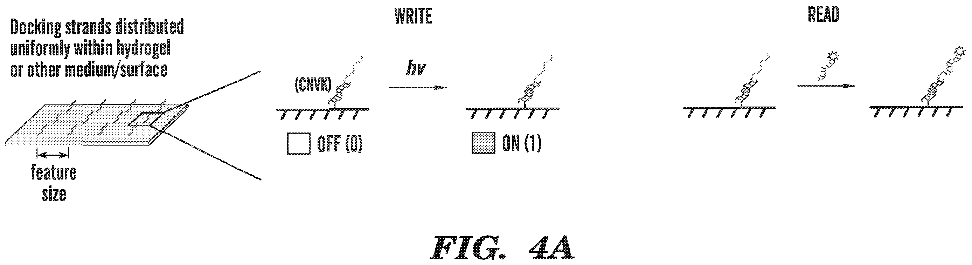

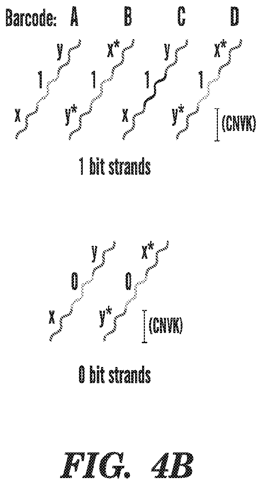

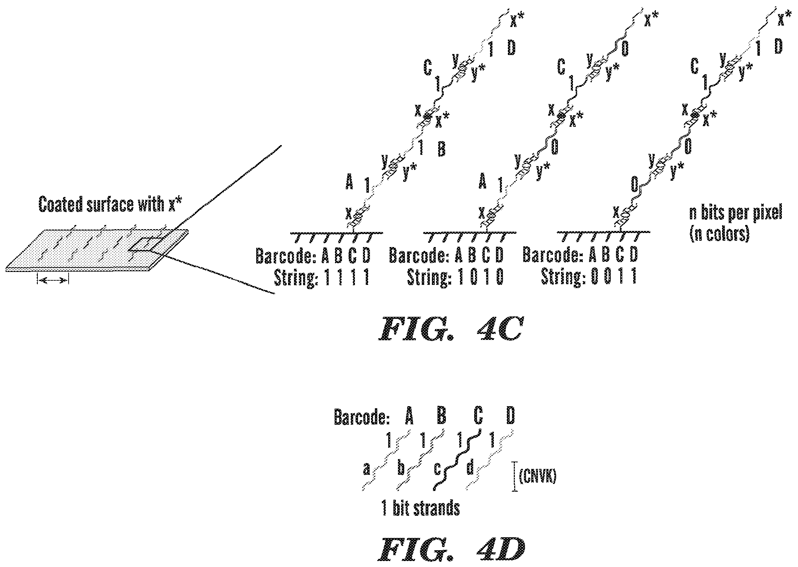

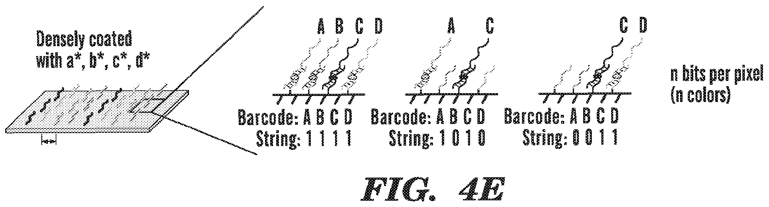

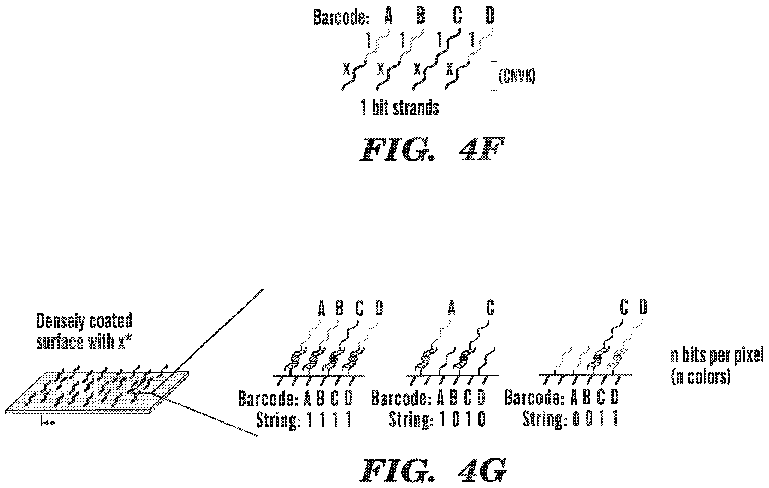

**FIGS. 4A-4G** depict methods for encoding bits in an area (*i.e.,* a pixel). **FIG. 4A** shows that docking strands can be uniformly or randomly embedded in the hydrogel, and the WRITE/READ operations as depicted in FIG. 3 can be used to change the state of the docking strand from OFF (0) to ON (1). A pixel is a defined area of the hydrogel onto which UV may or may not be applied. **FIG. 4B** shows that multiple 1 and 0 crosslinking bit strands can be designed to further increase the bit content per pixel. Depicted is an example of strands required for n = 4 bits per pixel (4 colors). **FIG. 4C** shows that using successive rounds of binding 1 bit strands, crosslinking specific pixels, washing, and binding 0 bit strands, concatemers representing multiple bits can be assembled. Three examples of 4-bit pixel concatemers that might be formed are depicted. **FIG. 4D** depicts another strategy for increasing bit content per pixel, which is to use multiple orthogonal bit strands. **FIG. 4E** shows that these orthogonal strands can bind to orthogonal docking strands embedded in the gel, and through successive bind and wash steps can be specifically bound to each pixel (ON state 1) or not (OFF state 0). In this schematic, three possible 4-bit pixel configurations are shown. **FIG. 4F** shows barcode strands designed to hybridize on the same binding domain (x*) that has been densely embedded in the surface. **FIG. 4G** show that by programming the binding reaction kinetics (*e.g*. short time, low barcode strand concentration) a minority of binding sites may be occupied by barcode strands of interest at the time of cross-linking, so that the same binding sequence can be recycled for all barcode sequences.

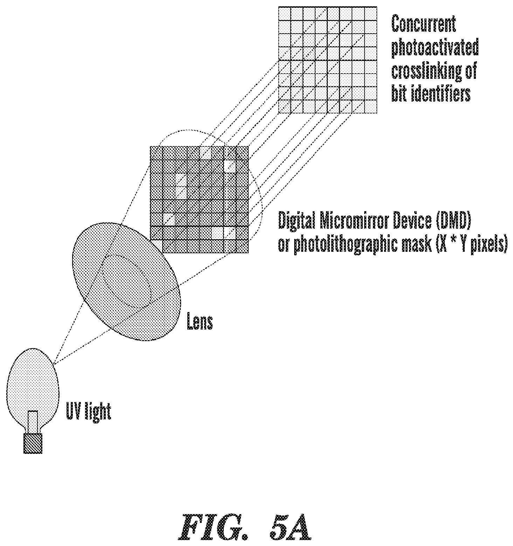

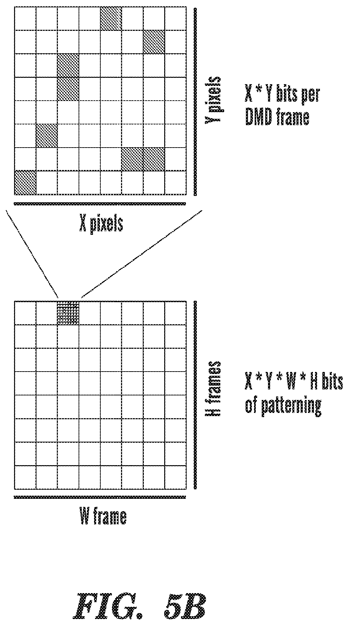

**FIGS. 5A-5B** demonstrate the utility of Digital Micromirror Devices (DMDs) for high throughput pixel patterning. **FIG. 5A** shows that a UV light source (365nm) can be passed through a DMD, or photolithographic mask, to illuminate only specific pixels within its frame, thereby crosslinking strands only within those specific pixels. **FIG. 5B** shows that multiple frames can be patterned in a multiplexed fashion, producing a larger scale pattern.

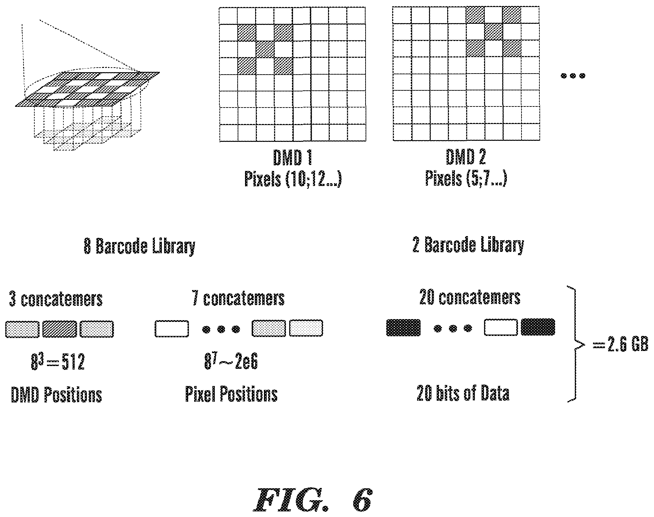

**FIG. 6** depicts an encoding strategy with barcode concatemers. Each pixel illuminated by a DMD contains DNA data barcode concatemers of up to 30-40 units in length.

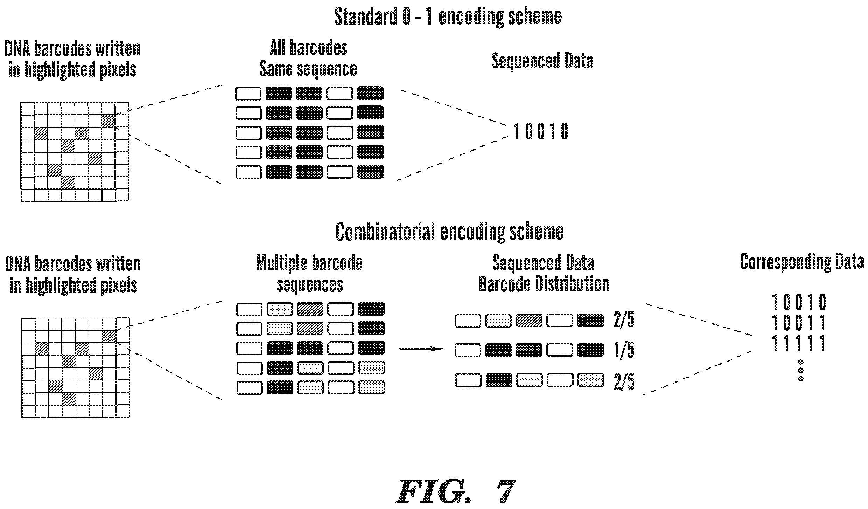

**FIG. 7** depicts a schematic for increasing data density with combinatorial barcoding. In the combinatorial encoding scheme, quantitative information regarding distributions of barcode sequences can be used for deconvolution of the actual data string based on expected relative frequencies of sequences.

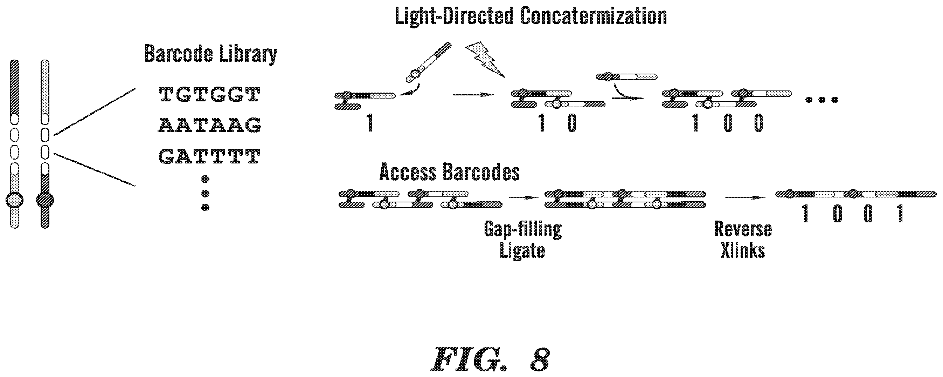

**FIG. 8** demonstrates exemplary methods for copying and retrieving barcode information. A DNA data barcode set is shown on the left, containing alternating hybridization domains (filled lines), a barcode sequence (dotted lines), and a photoreactive element (filled circle). During light-directed concatemerization (top right), each DNA data barcode will be hybridized iteratively to form a long chain concatemer. Barcodes can be accessed by filling in gaps with gap-filling polymerases, ligation and reversal of crosslinks (bottom right).

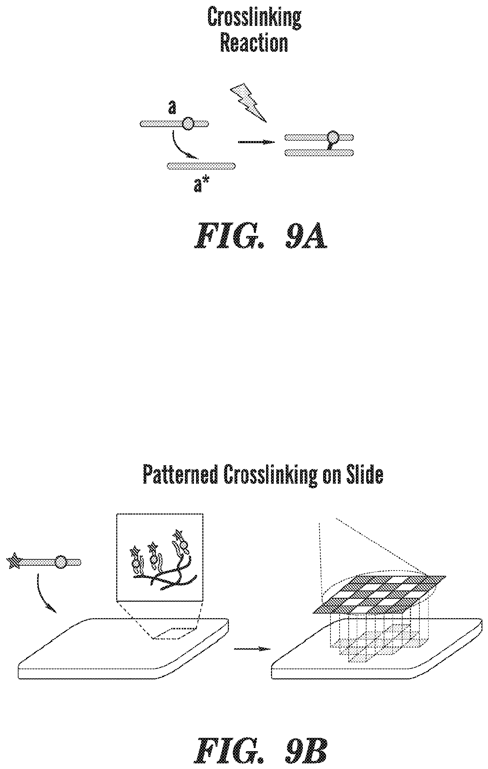

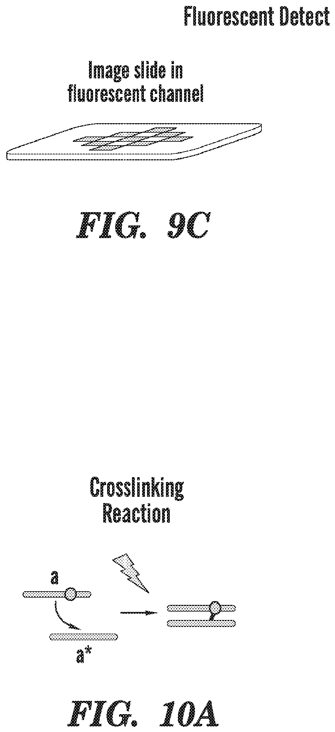

**FIGS. 9A-9C** depict experimental validation of DMD patterning of DNA on a solid surface. **FIG. 9A** shows a strand with sequence containing a CNVK modification (circle), capable of crosslinking with a hybridized strand upon exposure to UV light. **FIG. 9B** shows the surface of a glass slide that has been functionalized with BSA-biotin, streptavidin and biotinylated-sequence a*. Fluorophore-labeled CNVK sequences were then bound to these docking sequences and exposed to 405 nm light using a DMD to provide a checkerboard illumination profile. **FIG. 9C** shows the resultant checkerboard pattern on the glass slide after imaging in the fluorescent channel with a Typhoon fluorescent scanner.

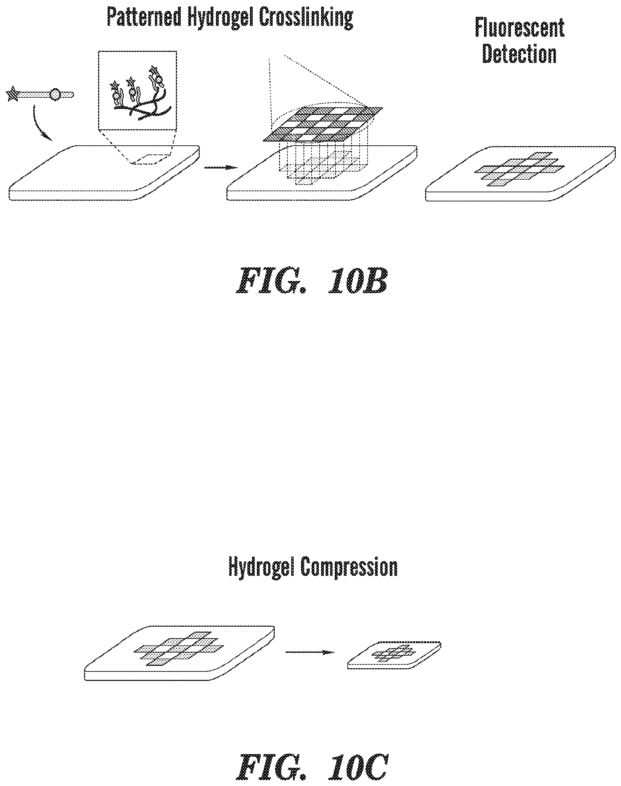

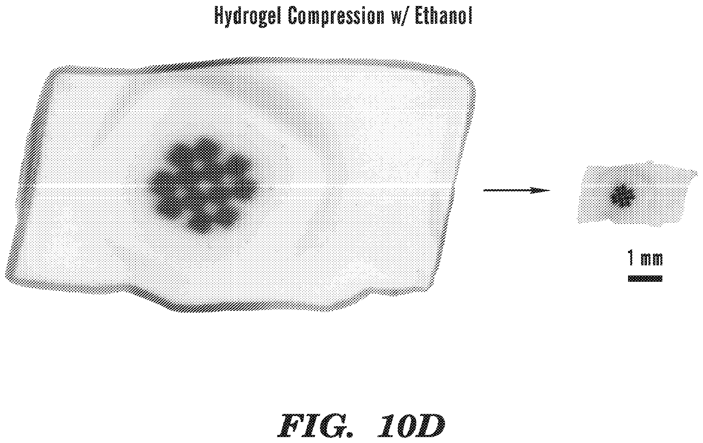

**FIGS. 10A-10D** depict experimental validation of DMD patterning on compressible hydrogels. **FIG. 10A** shows a strand with sequence a contains a CNVK modification (circle), capable of crosslinking with a hybridized strand upon exposure to UV light. **FIG. 10B** shows a compressible hydrogel containing docking strands (sequence a*) that were acrydite modified and polymerized into the gel. After binding to crosslinking strands, a DMD was used to illuminate a checkerboard pattern on the gel. **FIG. 10C** shows that hydrogels can be physically compressed using a range of conditions (*e.g.* low salt, ethanol incubation). **FIG. 10D** shows fluorescent images of the hydrogel before and after physical compression side-by-side. The DNA-based checkerboard pattern is maintained through the compression process.

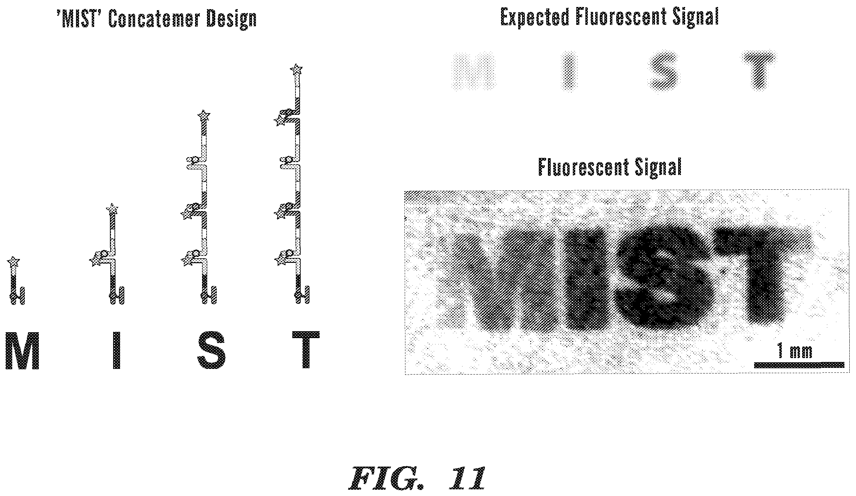

**FIG. 11** depicts a barcoding experiment using concatemerized DNA in connection with a DMD to spell out the letters 'MIST' onto a slide.

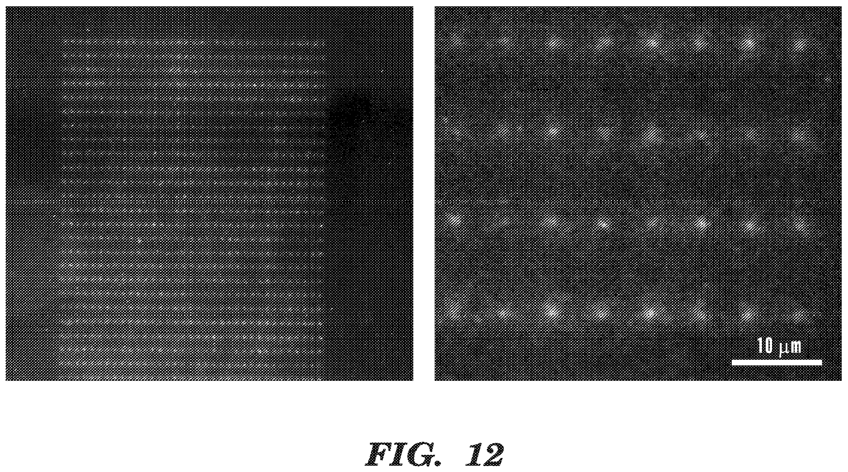

**FIG. 12** shows patterned crosslinking on a glass surface with fluorescent crosslinking strands demonstrating 1 micrometer sized features. Crosslinking was performed with a 1 second UV pulse. The slide was then scanned in the TRITC channel with a 40x objective.

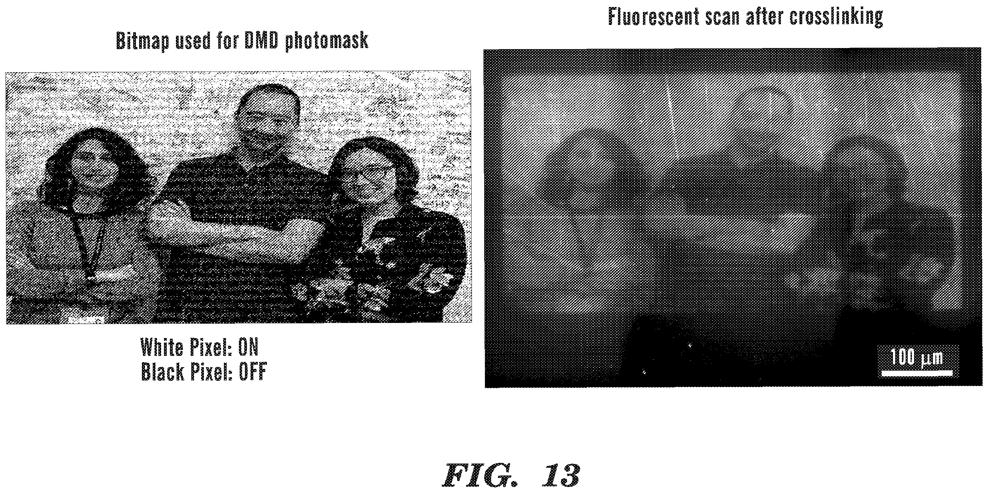

**FIG. 13** demonstrates the pixel level spatial control of photopatterning on a glass slide. (Left) Bitmap (600 X 680) used for the DMD photomask, a white pixel flips the corresponding micromirror in the DMD 'ON' and vice versa for a black pixel. (Right) Crosslinking of a fluorescent crosslinker strand was performed on a glass slide for 1 second. The crosslinking spatial profile was focused through a 10x objective (~1um feature size) onto a glass slide then scanned in the TRITC channel with a 40x objective.

### DETAILED DESCRIPTION

Generally, the methods provided herein are based in part, on the discovery of methods and compositions that allow for high-throughput concatemerization of nucleic acids and the production of combinatorial sequences with spatial information and/or data. The methods and compositions described herein are useful in many applications, such molecular coding, data storage, tissue engineering, communication, and biosensors.

As used herein, the term "barcode strand" generally refers to a single-stranded nucleic acid that is 5-10 nucleotides in length and encodes data. In some embodiments, a barcode strand is 5, 6, 7, 8, 9, or 10 nucleotides in length. In some embodiments, a barcode strand may be more than 10 nucleotides in length. In some embodiments, a barcode strand may be assigned a bit value of 0 or 1. In some embodiments, a barcode strand may be read using a nucleic acid sequencing technology. In some embodiments, the sequence of the barcode strand may be determined through the use of complementary sequences labeled with detectable moieties such as fluorophores, quantum dots, peptide tags, beads (*e.g*., agarose, latex, magnetoresponsive, chromatic), polymer dots, nanoparticles, additional docking sites, tags such as biotin, or functional groups such that their presence may be detected *e.g.,* by fluorescence microscopy, fluorescent scanners, optical scanners and the like.

As used herein the term "complementary" generally refers to the potential for a hybridized pairing or binding interaction between two sets of nucleic acids. Complementary nucleic acids are capable of binding to one another through hydrogen bond pairing according to canonical Watson-Crick base pairing and non-Watson-Crick base pairing (e.g., Wobble base pairing and Hoogsteen base pairing). In some embodiments, two sets of nucleic acids may be 100% complementary to one another. In other embodiments, two sets of nucleic acids may comprise 1, 2, 3, 4, 5, 6, 7, 8, 9, 10, or more nucleotides that are not complementary. In other embodiments, two sets of nucleic acids may be at least 50%, at least 60%, at least 70%, at least 80%, or at least 90% complementary. In some embodiments, two sets of nucleic acids are complementary so long as they are capable of forming a stable or transient complex. As used herein, the term "complementary strand" generally refers to a single-stranded nucleic acid that is 200 nucleotides or fewer in length. In some embodiments, a complementary strand may be 5-100, 5-75, 5-50, 5-25, 5-15, or 5-10 nucleotides in length. In some embodiments, a complementary strand may be 10-100, 10-50, 10-25, 10-20, 15-25, or 15-25 nucleotides in length. In some embodiments, a complementary strand may be 7 nucleotides in length. In some embodiments, a complementary strand comprises at least one detectable moiety. In some embodiments, a detectable moiety may be a fluorophore or a nanoparticle. In some embodiments, a complementary strand is complementary to a crosslinking strand, optionally wherein the crosslinking strand is a component of a first nucleic acid barcode and the complementary strand is a component of a second nucleic acid barcode. In some embodiments, a crosslinking strand is DNA or RNA.

As used herein, the terms "compressible hydrogel", "hydrogel" and "nucleic acid-encoded hydrogel" are used interchangeably and generally refer to any polymeric material that is capable of retaining a significant fraction of water within its structure without dissolving into an aqueous solution. A compressible hydrogel may be any polymeric material produced by a reaction of one or more monomers that is capable of existing in a compressed state and an expanded state depending on its surrounding environment. Further, a compressible hydrogel is capable of being encoded with a nucleic acid pattern.

As used herein, the term "crosslinking strand" generally refers to a single-stranded nucleic acid that is 200 nucleotides or fewer in length. In some embodiments, a crosslinking strand may be 5-100, 5-75, 5-50, 5-25, 5-15, or 5-10 nucleotides in length. In some embodiments, a crosslinking strand may be 10-100, 10-50, 10-25, 10-20, 15-25, or 15-25 nucleotides in length. In some embodiments, a crosslinking strand may be 7 nucleotides in length. In some embodiments, a crosslinking strand comprises at least one photoreactive nucleotide. In some embodiments, a crosslinking strand comprises at least one CNVK or CNVD nucleotide. In some embodiments, a crosslinking strand is complementary to a complementary strand, optionally wherein the crosslinking strand is a component of a first nucleic acid barcode and the complementary strand is a component of a second nucleic acid barcode. In some embodiments, a crosslinking strand is DNA or RNA. In some embodiments, crosslinking strands are further functionalized with moieties such as fluorophores, quantum dots, biotin, streptavidin, functional chemical groups, and other tags or nanoparticles.

As used herein, the term "docking strand" generally refers to a single-stranded nucleic acid that is 200 nucleotides or fewer in length and is associated with, *e.g.,* bound to, a compressible hydrogel. In some embodiments, a docking strand comprises a functional group, *e.g.,* an acrydite modification, that enables the docking strand to covalently attach or link to the hydrogel. A docking strand may comprise a docking site that is complementary to a complementary domain of a crosslinking strand. In some embodiments, a docking strand is 100 nucleotides or fewer. In some embodiments, a docking strand is 50 nucleotides of fewer. In some embodiments, a docking strand binds to an imager strand. In some embodiments, a docking strand is DNA or RNA. In some embodiments, docking strands may be attached to a surface such as glass, polystyrene, paper or silicon. In some embodiments, docking strands contain biotin or streptavidin modifications to mediate their binding to surfaces. In some embodiments, docking strands may be conjugated or otherwise bound to magnetic particles, gold nanoparticles, glass beads, streptavidin beads, streptavidin resin, antibodies, nitrocellulose membranes, paper, glass fiber membranes, or any solid-support columns.

As used herein, an "attachment nucleic acid strand" refers to any nucleic acid that allows for the nucleic acids described herein to associate with, crosslink to, embed into, or tether to, covalently or non-covalently interact with the substrate described herein. In some embodiments, the attachment nucleic acid strand comprises a barcode domain and a hybridization domain, wherein the hybridization domain optionally comprises a photoreactive element. In some embodiments, the attachment nucleic acid strand is substantially complementary to at least part of the first nucleic acid.

As used herein, the term "hybridization domain(s)" generally refers to either a crosslinking strand or a complementary domain. In some embodiments, a hybridization domain is a crosslinking strand, as defined herein. In some embodiments, a hybridization domain is a complementary strand, as defined herein. In some embodiments, two alternating hybridization domains refer to a single crosslinking strand and a single complementary strand.

As used herein, the term "nucleic acid barcode" generally refers to a single-stranded nucleic acid that comprises at least two of the following: a crosslinking strand, a barcode strand, and/or a complementary strand. If a nucleic acid barcode comprises a barcode strand, it comprises the data contained within its associated barcode strand. In some embodiments, a series of nucleic acid barcodes, *e.g.,* at least three nucleic acid barcodes, may be written into a nucleic acid concatemer. In some embodiments, a nucleic acid barcode or a set of nucleic acid barcodes may be patterned onto a substrate support, *e.g.,* a compressible hydrogel. In some embodiments, a nucleic acid barcode may be at least 15, 20, 25, 30, 40, 50, 60, 70, 80, 90, or 100 nucleotides in length. In some embodiments, a nucleic acid barcode is 20 nucleotides in length. In some embodiments, a nucleic acid barcode comprises a barcode strand that is 6 nucleotides in length, a crosslinking strand that is 7 nucleotides in length, and a complementary strand that is 7 nucleotides in length. A nucleic acid barcode generally comprises the following domain structure in the 5' to 3' direction: 5'-Crosslinking strand-to-Barcode strand-to-Complementary strand.

As used herein, the term "barcode domain" or "DNA data domain" refers to the part of the barcode strand that comprises a nucleic acid sequence that represents special information, an arbitrary value, or code. The barcode domain sequence can be predetermined by a barcode library. The barcode domain can be a sequence comprising DNA, RNA, synthetic nucleobases, or any combination thereof.

As used herein, the term "barcode library" is a collection of stored nucleic acid sequences with associated information. Each sequence and the associated information are stored in a database with information such as the sequence, pattern, structure, and label. The barcode library can be used to decipher or read the special information contained in each barcode strand. The barcode library can also be used to pre-determine the concatemer pattern for data storage, writing, and reading of the concatemers.

As used herein, the term "nucleic acid concatemer" generally refers to a nucleic acid that comprises at least three nucleic acid barcodes. A nucleic acid concatemer may comprise nucleic acid barcodes that are covalently linked to one another via photoreactive nucleotides. In some embodiments, a nucleic acid concatemer may comprise at least 1, at least 2, at least 3, at least 4, at least 5, or at least 10 nucleic acid barcodes. In some embodiments, a nucleic acid concatemer may comprise at least 1, at least 2, at least 3, at least 4, at least 5, or at least 10 barcode strands that each incorporate data, *e.g.,* each barcode strand may uniquely/independently be assigned to a bit value.

As used herein, the term "bit value" refers to an arbitrary value that can be used to identify a position along x, y, and z coordinates; an integer; a symbol; a character; or a letter. In computing, bit numbering is the convention used to identify the bit positions. The bit value can be a binary number associated with the binary numeral system. The binary numeral system uses two symbols: zero "0" and one "1", that represent a positional notation. Many 1s and 0s can be strung together to represent larger numbers. In the context of the barcodes provided herein, the bit value is assigned to a barcode domain and can be identified using a barcode library. The bit values can be used to store data and special information. For example, see FIGs. 4A-4G, and FIG. 6, and FIG. 7.

As used herein, the term "pattern" generally refers to any recognizably distinct image, structure, or design. A pattern, in some embodiments, is a geometric shape, such as a square, circle, or triangle. A pattern may contain repeating elements. A pattern may contain arbitrary elements. In some embodiments, a pattern is asymmetrical. In some embodiments, a pattern is symmetrical. The pattern can comprise spatial information and/or special information (*e.g.,* images, pixels, arbitrary values, and codes).

As used herein, the term "special information" is any data or spatial information that can be stored in the barcode. Non-limiting examples of special information include letters, numbers, text, coordinates, images, graphics, movies, sequencing data, QR codes, binary codes, passcodes, and health records.

As used herein, the term "spatial information" is any information, coordinates, markers in a biological tissue or matrix, that can be stored in the barcode. The spatial information can inform one of skill in the art where on the substrate a particular marker, barcode, or pattern is located. For example, spatial information may be useful in creating an image or QR code with the nucleic acid barcodes. Spatial information can also be useful in the sensing function of the nucleic acid barcode in the presence of a barcode-revealing agent as provided herein.

As used herein, the term "agent" refers to any substance, chemical constituent, chemical molecule of synthetic or biological origin.

As used herein, the term "plurality" generally refers to any number or value greater than one. A plurality may be at least 2, at least 3, at least 5, at least 10, at least 20, at least 25, at least 30, at least 40, at least 50, at least 100, at least 150, at least 200, at least 250, at least 300, at least 350, at least 400, at least 500, or at least 1,000. A plurality of nucleic acids such as a plurality of nucleic acid staple strands or a plurality of SSTs may comprise at least 2, at least 3, at least 5, at least 10, at least 20, at least 25, at least 30, at least 40, at least 50, at least 100, at least 150, at least 200, at least 250, at least 300, at least 350, at least 400, at least 500, or at least 1,000 nucleic acids. In some embodiments, a plurality of nucleic acids includes 2-1000, 5-1000, 10-1000, 50-1000, 100-1000, or 500-1000 nucleic acids. In some embodiments, a plurality of nucleic acids includes 2-5000, 5-5000, 10-5000, 50-5000, 100-5000, or 500-5000 nucleic acids.

### Light-directed data writing with nucleic acids

Methods for light- directed concatemerization of barcodes (e.g., DNA data barcodes) are provided herein (FIG. 1). Each nucleic acid barcode strand can be about 5-10 bases in length. In some embodiments, a nucleic acid barcode strand can be 4, 5, 6, 7, 8, 9, or 10 bases in length. In some embodiments, a nucleic acid barcode strand can be about 10 or about 10-15 bases in length. Individual unique barcode strand can be assigned a bit value of '0' or '1'. Thus a concatenated string of nucleic acid barcodes can be equivalent to a string of 0's and 1's (i.e. digital information) (FIG. 1D, FIG. 1E).

Barcode strands, or barcode sequences, are designed for robust data retrieval, optionally for use with imperfect sequencing devices. An example barcode library shown has a minimum of Hamming distance 4, meaning that at least 4 substitution errors must be made by a sequencing device before barcode aliasing occurs. Other variations in error correcting codes can also be used, including redundancy sequences, parity sequences, or equivalents can also be incorporated into the barcode sequence (FIG. 1A).

The basic design of a nucleic acid barcode will comprise of the barcode strand *(e.g.,* about 5-10 bases in length), flanked by a set of two alternating hybridization domains. This will allow any arbitrary arrangement of barcodes through iterative hybridization, crosslinking and washing steps (FIG. 1A). In some embodiments, a hybridization domain can be a crosslinking strand. In some embodiments, a hybridization domain can be a complementary strand. In some embodiments, a set of two alternating hybridization domains can comprise one crosslinking strand and one complementary strand. In some embodiments, a nucleic acid barcode comprises a barcode strand linked to (i) a crosslinking strand that comprises a photo-activated nucleotide and (ii) a complementary strand. In some embodiments, the alternating hybridization domains can be at least 5, at least 10, at least 15, or at least 20 bases in length. In some embodiments, the alternating hybridization domains comprise enough bases to provide capability to hybridize to a hybridization domain of a second nucleic acid barcode. In some embodiments, a crosslinking strand of one nucleic acid barcode will hybridize with a complementary strand of another.

Nucleic acids, *e.g.,* DNA data barcodes, can be concatenated and covalently fixed together by a photo-chemical crosslinking reaction. The crosslinking reaction itself is light driven and can be performed in aqueous solution. Crosslinking strands of nucleic acid barcodes comprise at least one photo-reactive nucleobase. In some embodiments, the photo-reactive nucleobase can be any modified nucleobase that is capable of forming a crosslink with another nucleobase in the presence of light. In some embodiments, the photo-reactive nucleobase can be a modified pyrimidine or purine nucleobase. In some embodiments, the photo-reactive nucleobase can be 3-Cyanovinylcarbazole Phosphoramidite (CNVK) or 3-Cyanovinylcarbazole modified d-threoninol (CNVD). In some embodiments, the photo-reactive nucleobase can comprise a vinyl, acrylate, N-hydroxysuccinimide, amine, carboxylate or thiol chemical group. In some embodiments, the photo-reactive nucleobase comprises a bromo-deoxyuridine. The CNVK crosslinking base displays highest crosslinking efficiency with a thymine (T) base that is positioned adjacent to the base on the complementary strand [10] and can be directly incorporated into the DNA hybridization domain itself as a base substitution (FIG. 1B). In some embodiments, a crosslinking reaction is performed using 365 nm wavelength of light and can be completed within 1 second (FIG. 1C). In some embodiments, a crosslinking reaction can be performed using any wavelength of visible or ultraviolet light. In some embodiments, a crosslinking reaction can be completed within 0.1, 0.25, 0.5, 1, 5, or 10 seconds. In some embodiments, a crosslinking reaction can be completed within 0.5, 1, 5, 10, 20, 30, 40, 50, or 60 minutes. In some embodiments, a crosslinking reaction has negligible effects on bases that neighbor the photoreactive nucleobase. In some embodiments, other photochemical nucleic acid crosslinking agents, including psoralen can be used in combination with nucleic acid barcodes.

In some embodiments, CNVK-labeled strands can be hybridized to docking strands by 1 to 10 bases or by 10 to 20 bases, under salt and temperature conditions such that they bind only for less than 1 second, between 1 and 10 seconds, or for less than 1 minute on average. In other embodiments, CNVK-labeled strands can have increased homology domains to docking strands, (20-40 bases, more than 40 bases), salt can be increased, or temperature decreased so that they are considered stably bound as they on average remain bound for longer than one minute. In general, conditions can be adjusted so that the CNVK-labeled strands have the desired average bound time to docking strands (t_bound = 1/k_off).

Concatemers of nucleic acid barcode can comprise at least two, at least three, at least four, at least five, at least ten, at least 15, at least 20, at least 25, at least 30, or at least 50 nucleic acid barcodes.

The concatemers of nucleic acid barcodes can be in various positions on the substrate provided herein. For example, concatemers can be linear, have two different orientations, or multiple orientations in different directions. The barcodes can be positioned along x, y, and z coordinates in space.

In some aspects, described herein is a barcode composition comprising:
a. a first nucleic acid comprising a barcode domain flanked by a pair of hybridization domains;
b. a second nucleic acid comprising a barcode domain flanked by a pair of hybridization domains;
c. a substrate;
d. optionally, an agent, wherein the agent permits attachment of the first nucleic acid to the substrate;
e. optionally, at least one additional nucleic acid that comprises a barcode domain flanked by a pair of hybridization domains;

wherein each barcode domain is assigned an independent barcode,
wherein at least one of the hybridization domains of each nucleic acid comprises a photoreactive element,
and wherein at least one nucleic acid is immobilized to the substrate at a predetermined location.

In some embodiments, the photoreactive element is a photoreactive nucleotide. In some embodiments, the photoreactive nucleotide is a CNVK or CNVD crosslinking base. In some embodiments, the photoreactive element is psoralen.

In some embodiments, the barcode domain of the first and/or second nucleic acid is selected from a barcode library having a minimum Hamming distance of 4.

In some embodiments, at least one nucleic acid comprises a label. In some embodiments, the label is a fluorophore.

In some embodiments, the substrate is selected from the group consisting of: glass, transparent polymers, polystyrene, hydrogels, metal, ceramic, paper, agarose, gelatin, alginate, dextran, iron oxide, stainless steel, gold, copper, silver chloride, polycarbonate, polydimethylsiloxane, polyethylene, acrylonitrile butadiene styrene, cyclo-olefin polymers, cyclo-olefin copolymers, streptavidin, resin, and a biological material. In some embodiments, the substrate is a compressible hydrogel. In some embodiments, the biological material is selected from the group consisting of: a tissue, a cell, an organoid, an engineered tissue; and an extracellular matrix.

In some embodiments, the barcode composition further comprises a concatemer of nucleic acids comprising a photoreactive element.

In some embodiments, the concatemer of nucleic acids encode special information and/or spatial information.

In some embodiments, the special information is selected from the group consisting of: text, images, coordinates, graphics, movies, sequencing data, QR codes, binary codes, and health records.

In some embodiments, the nucleic acids are covalently linked to at least one other of the nucleic acids through a single photoreactive element of a hybridization domain.

In some embodiments, each barcode domain comprises at least 5 nucleotides, optionally 5 to 10 nucleotides.

In some embodiments, one or more nucleic acids are attached to the substrate in a predetermined pattern. In some embodiments, the predetermined pattern is a geometric shape, a square, a circle, or triangle. In some embodiments, the predetermined pattern comprises repeating elements.

In some embodiments, the predetermined pattern is asymmetrical or symmetrical. In some embodiments, the predetermined pattern comprises spatial information and/or special information. In some embodiments, the first nucleic acid is attached at a 0.5 - 5 micron, optionally a 1 micron, resolution. In some embodiments, the first nucleic acid is attached at a 0.25 - 100 micron, optionally a 1 micron, resolution.

In some embodiments, the agent for attachment of the first nucleic acid to the substrate is an attachment nucleic acid strand; and wherein the attachment nucleic acid strand is substantially complementary to at least part of the first nucleic acid.

In some embodiments, the agent for attachment of the first nucleic acid to the substrate is an attachment nucleic acid strand comprising a barcode domain and a hybridization domain, wherein the hybridization domain optionally comprises a photoreactive element.

### Substrates

Nucleic acid barcodes or concatemers of nucleic acid barcodes can be applied to any substrate surface, without the need for specialized surface treatment, such as formation of microwells common in microarray chips. Surfaces only require functionalization with nucleic acid strands which will serve as the initial docking strand of a nascent chain barcode concatemer. Alternatively, the nucleic acids can form non-covalent interactions with the substrate.

As used herein, the terms "substrate" or "substrate surface" are used interchangeably to describe a structure upon which one or more nucleic acid barcodes or concatemers of nucleic acid barcodes provided herein can be displayed or in contact with for contact with additional nucleic acids and/or labels. The nucleic acid barcodes provided herein can be conjugated to the substrate surface.

As used herein, the term "conjugated to" encompasses association of a nucleic acid with a substrate surface, a phase-changing agent or a member of an affinity pair by covalent bonding, including but not limited to cross-linking via a cross-linking agent, or by a strong non-covalent interaction that is maintained under conditions in which the conjugate is to be used.

As used herein, the term "hybridize" refers to the phenomenon of a single-stranded nucleic acid or region thereof forming hydrogen-bonded base pair interactions with either another single stranded nucleic acid or region thereof (intermolecular hybridization) or with another single-stranded region of the same nucleic acid (intramolecular hybridization). Hybridization is governed by the base sequences involved, with complementary nucleobases forming hydrogen bonds, and the stability of any hybrid being determined by the identity of the base pairs (*e.g.,* G:C base pairs being stronger than A:T base pairs) and the number of contiguous base pairs, with longer stretches of complementary bases forming more stable hybrids. For example, hybridization between docking strands and nucleic acid barcodes comprising a photo-reactive nucleobase, *e.g.,* CNVK base, permit the light-directed reading and/or visualization of the data stored on the substrate surface.

The substrate surface provided herein can exist in the form of a biological material (*e.g.,* cell, tissue, or fragments thereof), platform, column, filter or sheet, dish, a microfluidic capture device, capillary tube, electrochemical responsive platform, scaffold, cartridge, resin, matrix, bead, phase changing agent, or another substrate surface known in the art. Multiple surface types can be used. Non-limiting examples of substrate surfaces include glass, transparent polymers, polystyrene, hydrogels, metal, ceramic, paper, agarose, gelatin, alginate, dextran, iron oxide, stainless steel, gold nanobeads or particles, copper, silver chloride, polycarbonate, polydimethylsiloxane, polyethylene, acrylonitrile butadiene styrene, cyclo-olefin polymers or cyclo-olefin copolymers, streptavidin, Sepharose^{™} resin, biological materials (*e.g.,* cells, tissues, cell membranes, extracellular matrix proteins, etc.), and combinations thereof.

In some embodiments, the substrate can be a glass or polymer surface. In some embodiments, the substrate is a compressible hydrogel.

In some embodiments, the biological material is selected from the group consisting of: a tissue, a cell, an organoid, an engineered tissue; and an extracellular matrix.

In some embodiments, nucleic acid barcodes or concatemers of nucleic acid barcodes can be applied to, or embedded within, a compressible hydrogel. In some embodiments, nucleic acid barcodes or concatemers of nucleic acid barcodes represent special information, *e.g.,* digital data and can store any information, including but not limited to text, images, graphics, movies, sequencing data, and/or health records. In some embodiments, the nucleic acid barcodes or concatemers of nucleic acid barcodes represent spatial information.

Methods of surface functionalization of these substrates with nucleic acid strands is known in the art and requires few material requirements and minimal preparation time. A typical preparation first involves passivating the surface with Bovine Serum Albumin-biotin (BSA-Biotin). The BSA binds nonspecifically with the glass surface. Secondly, a streptavidin protein will bind to the biotin attachment on the BSA protein. Finally, a biotin labeled nucleic acid can be introduced to bind to the other available binding sites on the streptavidin protein, completing the functionalization of the glass surface.

Functionalization of the substrate surface with the initial nucleic acid docking strand (FIG. 3, blue strand) will vary depending on the surface type. Functionalization of glass surfaces is as described above. For substrate surfaces, docking strands can be incorporated directly into the substrate or hydrogel matrix itself during the polymerization step. Acrydite modified nucleic acid strands can be mixed with the substrate or hydrogel material and be polymerized along with the substrate or hydrogel material.

In some embodiments, biological materials such as molecules, cell-free reactions, cells, tissue sections, organoids and organisms can be immobilized on the substrate described herein. Barcoded surfaces and substrates can be pre-patterned with a known configuration of spatial barcodes. Barcoded surfaces can be used as a grid for spatial barcoding of the biological material. Substrates can serve as docking sites for various targets in biological samples, including genomic and ribonucleic targets. Docking sites on barcoded substrates can carry functional groups, including chemical or protein tags, that can be used to bind to protein, metabolic or other targets in biological materials. Optionally, nucleic acid barcodes on the barcoded substrate can be cleaved off from the surface, using chemical, enzymatic, or photochemical methods and transferred to the biological material through diffusion or electrophoresis, force spectroscopy, or magnetic fields while preserving the overall barcode pattern.

### Compressible Hydrogels

Compressible hydrogels are three-dimensional (3D) polymer networks that comprise high water content (up to 99 % of the hydrogel mass) [3]. This gives hydrogels the ability to considerably expand and compress (>10 times in volume) in response to the amount of water in the polymer network, which can be modulated via environmental stimuli such as ionic strength, pH temperature, light, electric and magnetic fields, solvent composition, and pressure. Hydrogels have been used widely in biomedicine (*e.g.,* in drug delivery, contact lenses, tissue engineering, biosensing, photodynamic therapy), microtechnology (*e.g.,* in actuators, supercapacitors), industry, and microscopy [4].

The size of hydrogels can be modulated based on varying environmental conditions such as salt concentration, solute concentration, temperature, pH, and presence of nucleic acids [5], [6]. It has been demonstrated that embedding of features into hydrogels allows for the expansion of the distance between features of interest from below the diffraction limit to above it, enabling super-resolution imaging through physically expanding the gel [7]. A key feature of compressible hydrogels is that the hydrogel compression and expansion can be obtained isotropically, such that features maintain their relative spatial distances from each other regardless of hydrogel size. Furthermore, hydrogels can be easily molded, patterned, or shaped into any shape, size, or form, according to the application for use of the hydrogel or visual readout. The hydrogels provided herein can be multidimensional (*e.g,* 2D, 3D or swellable (4D)) hydrogels. See for example, McCracken, J. et al. 2016. "Programming Mechanical and Physicochemical Properties of 3D Hydrogel Cellular Microcultures via Direct Ink Writing." Advanced Healthcare Materials 5: 1025-1039; Gladman, et al. 2016. "Biomimetic 4D Printing." Nature Materials 15: 413-419; Ramon-Azcon et al. Lab on a Chip (2012); US Application No. 9,410,267 B2; and US Patent No. 8.999,378 B2.

Hydrogels can also be cured on the surface of many other materials (e.g., polymers, glass, or plastics). For example, it is known in the art that hydrogels can be used as scaffolds for biological patterning, such as the development of 3D organoids and laminar tissues that mimic the *in vivo* microenvironment. It is contemplated that the nucleic acids provided herein can be embedded in any type of hydrogel or biologic in any form or pattern including engineered matrices and biological tissues or cellular arrangements. The nucleic acids provided herein can be used to program the arrangement of biological material or specifically identify or sort biological material for use in tissue engineering applications. The nucleic acids provided herein can be useful in constructing cell-cell interactions within an engineered tissue or organoid.

Hydrogel patterning can be in the form of any pre-determined pattern engineered using methods known in the art (*e.g.,* nanomolding, micromolding, microcontact printing, injection molding, masking techniques, photolithography methods, curing, maskless patterning, photosensitive hydrogel patterning, 3D printing, rotary jet spinning, and the like). Patterns can be isotropic or anisotropic. Patterns can be in the form of lines, circles, tubes, spheres, fibers, letters, numbers, dots, polygons, squares, matrix barcode *(e.g.,* QR code), binary code, or any other pattern known in the art. The pattern can be any size, shape, or form that permits visualization or reading of the pattern.

Hydrogels can have physical properties, *e.g.,* elastic modulus or porosity, that can be varied. Hydrogels can be porous or non-porous. Methods of altering hydrogel physical properties, *e.g.,* elastic modulus, are known in the art and can vary based on concentration of solute used, temperature, pressure, curing procedures, the type of aqueous solution used or mixtures of solutes, and incubation times in aqueous solutions.

Compressible hydrogels, also known as expandable hydrogels have recently been used for microscopic analysis to physically enlarge the size of the embedded sample in order to improve the separation of molecular stains for higher resolution [7]. For these applications, 4-5 fold linear expansions of acrylate-acrylamide co-polymer hydrogels have been demonstrated through the absorption of water in the presence of low-salt or salt-free solution. An expanded hydrogel can be re-embedded in another expandable gel and expanded again, to achieve < 20-fold linear expansion [8]. Alternatively, N,N-dimethylacrylamide acid (DMAA) crosslinked with sodium acrylate (SA) has been used to produce an expandable gel matrix that can undergo 10-fold linear expansion in a single step [9]. These gels can similarly be isotropically compressed through dehydration. This can be achieved by using solutions with high salt concentration or through replacement of water with other liquids such as ethanol or organic solvents.

Hydrogels can be formed of several different materials including water-soluble polymers such as poly(acrylic acid), poly(vinyl alcohol), poly(vinylpyrrolidone), poly(ethylene glycol), polyacrylamide, and polysaccharides. Alternatively, natural polymers such as gelatin, agar, dextran, or collagen can be utilized. Crosslinking can be performed through chemical means using a polymerization initiator, or through radiation or thermal treatments. In expanded form, hydrogels can withstand a high water content (reaching ~99% water).

A hydrogel can be naturally occurring, derived from a natural source, or derived from a synthetic source. A hydrogel can be any water-swollen and cross-linked polymeric material produced by a reaction of one or more monomers. A hydrogel can be a polymeric material that is capable of expanding to retain a significant fraction of water within its structure without dissolving into the aqueous solution. A hydrogel can also be any shrinkable material, *e.g.,* heat-shrinkable plastics, viscoelastic foam, memory foam.

Hydrogels can be derived from natural monomeric molecules (*e.g.,* glycosaminoglycans), hydrophilic materials (*e.g.,* methacrylates, electrolyte complexes, vinylacetates, acrylamides), or natural polymeric materials (*e.g.,* peptides, saccharides). Other suitable hydrogel compositions are as described in U.S. Patent No. 6,271,278, issued August 7, 2001, entitled "Hydrogel composites and superporous hydrogel composites having fast swelling, high mechanical strength, and superabsorbent properties". Hydrogels can be comprised of hydrophobic and/or hydrophilic materials, wherein hydrophobic materials are not physically attracted to water and hydrophilic materials are physically attracted to water.

In some embodiments, a hydrogel can be a homopolymer-based hydrogel, wherein the hydrogel is derived from a single monomeric species or molecule. In some embodiments, a hydrogel can be a copolymer-based hydrogel, wherein the hydrogel is derived from two or more different monomer species or molecules. In some embodiments, a copolymer-based hydrogel is arranged in a random, block, or alternating configuration, optionally along the backbone of one of the monomers. In some embodiments, a hydrogel can be a multipolymer interpenetrating polymer-based hydrogel, wherein the hydrogel is derived from at least two different, optionally crosslinked, polymer subunits. In some embodiments, a multipolymer interpenetrating polymer-based hydrogel comprises one polymer subunit that is a crosslinked and one polymer that is a non-crosslinked polymer subunit.

A hydrogel may be non-crystalline, semicrystalline, or crystalline. A hydrogel may or may not be covalently crosslinked. A hydrogel can be synthesized using chemical methods (*e.g.,* chemical crosslinking) or physical methods (*e.g.,* hydrophobic interactions). A hydrogel can be neutrally charged, net positively charged, or net negatively charged. In some embodiments, a hydrogel comprises positively charged groups and negatively charged groups. In some embodiments, a hydrogel can be amphoteric or zwitterionic.

In some embodiments, a hydrogel can be pre-cast into a gel, mold, or other embedding materials before encoding with nucleic acids. In some embodiments, a hydrogel can be cast into a gel, mold or other embedding materials after encoding with nucleic acids.

The synthesis of, manipulation of, and/or addition of nucleic acids or other molecular species to a hydrogel can be facilitated using external stimuli such as electric field, magnetic field, pressure, suction and capillary action. The hydrogels provided herein can be modified for use as a biosensor (*e.g.,* monitoring diseases, treating diseases with controlled drug release mechanisms, contact lenses, skin or mucosal tissue engraftments, or microarray disease detection). Modifications to hydrogels for use in tissue engraftments and cellular scaffolds are known in the art.

In some embodiments, microfluidics can be used to synthesize, manipulate, or add nucleic acids or other molecular species to a hydrogel

In some embodiments, a hydrogel exists in a compressed state, wherein the hydrogel is fully compressed or shrunken and water content of the hydrogel is decreased. In some embodiments, a hydrogel exists in an expanded state, wherein the hydrogel is fully expanded, enlarged, or swelled and water content of the hydrogel is increased. In some embodiments, a hydrogel can exist in an intermediate state between fully compressed and fully expanded. In some embodiments, a hydrogel is compressed or expanded in response to changes in external environmental conditions. In some embodiments, external environmental conditions can include physical and chemical conditions, wherein physical conditions include temperature, electric potential, light, pressure, and sound, and wherein chemical conditions include pH, solvent composition (*e.g.,* change in amount water, organic solvents), ionic strength, and small molecule solutes.

### Data compression with substrates

Herein, the compression and expansion properties of substrate or hydrogels are utilized for physically compressing information (FIGs. 2A-1C). In a typical data storage workflow as depicted in FIG. 2A, data is first written, or patterned (WRITE), onto a substrate or compressible hydrogel before being stored (STORE). Subsequently, the data can be accessed (READ), and a resetting (RESET) operation can optionally be performed before the data can be stored again.

Compressible hydrogels embedded with nucleic acid barcodes have the advantage of high data density via physical compression of the hydrogel while retaining the spatial information of the nucleic acid barcodes. In previously published forms of nucleic acid data storage, the data encoded in the nucleic acids were often in the form of several thousand non-contiguous DNA oligo strands, necessitating the inclusion of spatial barcode data in each DNA strand to reconstruct the bit order, thereby reducing overall data storage density [12].

Typically, a hydrogel is first isotopically expanded by any of the methods described previously in the literature (*e.g.* lowering the salt concentration), so that it can be encoded, *i.e.,* patterned, with nucleic acids as depicted in FIGs. 5A-5B. After writing and/or patterning, the hydrogel can be compressed back to a smaller size using an method opposite of what was used for expansion (*e.g.* increasing salt). For more stable storage, the gel can be fully desiccated before being stored.

To read the data on a written and/or patterned substrate or hydrogel (FIG. 2C), gels are typically re-hydrated if necessary and then expanded to such a size that they can be decoded (READ) before being reset (RESET) if necessary. At this point, the gel can subsequently be re-compressed and desiccated for further storage as depicted in the last steps of FIG. 2B.

These methods of data compression are scalable due to the few monomeric components necessary to prepare a substrate or hydrogel and are very effective in their ability to increase information storage density. By physically compressing the amount of space that bits of information takes up, the information density can be dramatically reduced. For example, if all sides of a square hydrogel are compressed by 10x each, this results in a 100x increase in information density when patterning is done in 2D, or a 1000x increase in information density when patterning is done volumetrically. As an example, a patterning that can store single bits at 1 micron resolution (feature size), with side compression of 20x, can result in the storage of 1 bit per 50³ nm³, or 1 petabyte of data per cubic centimeter.

A nucleic acid pattern or series of nucleic acid barcodes encoded on a substrate or hydrogel can be copied, transferred or edited. In some embodiments, the pattern or series of nucleic acid barcodes can be copied or transferred to another substrate or hydrogel. In other embodiments, the pattern or series of nucleic acid barcodes can be copied or transferred to a non-hydrogel material. In some embodiments, the pattern or series of nucleic acid barcodes can be copied, transferred or edited using chemical methods or physical methods, wherein the chemical methods can involve the use of an enzyme.

Methods of transferring are known in the art, such as electrotransfer or diffusion transfer. See for example, US Patent Nos. 4,840,714 A and 8,173,002 B2 .

For example, the barcoded pattern can be converted to its complementary barcode or copied in place by using polymerases. The barcodes or the barcode copies can also be released chemically, thermally or enzymatically from the substrate. The released strands can be transferred into substrate materials such as hydrogels or biomaterials through diffusion or electrophoresis, force spectroscopy, centrifugation, capillary flow, or magnetic fields while preserving the overall pattern. If the barcode was constructed in a hydrogel, this release can be done before or after compression.

Before the transfer process, the barcoded pattern can optionally be fixed to protect the organization via covalent attachment of the strands to each other through bridging nucleic acids or chemical crosslinker. This allows the re-use of the same barcode pattern for multiple pattern transfers.

In some embodiments, the barcoded pattern can be used for microfabrication or as a substrate for etching, masking, deposition of other materials.

### Writing nucleic acid-encoded substrates

A substrate or compressible hydrogel can be encoded with any writing technology that can spatially pattern nucleic acids while the substrate or hydrogel is in its expanded form.

An exemplary writing technology involves the introduction of docking strands into a substrate or hydrogel to function as a primary handle upon which to design a pattern. Docking strands, typically single-stranded nucleic acids of 200 nucleotides or fewer in length, can bind to, and form covalent crosslinks with a complementary domain of crosslinking strands. A plurality of docking strands can be embedded into the substrate or hydrogel by directly incorporating them into the substrate or hydrogel before the substrate or hydrogel materials are cast into a gel or mold. Alternatively, a plurality of docking strands can be encoded, e.g., patterned onto the hydrogel in a different manner (FIG. 4A). Docking strands can be randomly or uniformly distributed across the entirety of a substrate or hydrogel. In some embodiments, docking strands can be randomly or uniformly across a segment, or pixel, of a substrate or hydrogel. In some embodiments, the distribution of docking strands can generate a pattern. In other embodiments, the distribution of docking strands alone will not generate a pattern. A plurality of docking strands can be a plurality of identical docking strands with identical sequences. Alternatively, a plurality of docking strands can comprise 2, 3, 4, at least 5, at least 10, at least 15, or at least 20 different docking strands with unique sequences.

Nucleic acid barcodes, as described above herein, can then be attached to the docking strands through hybridization of a docking strand with the crosslinking strand of a nucleic acid barcode. Subsequent exposure to light may allow for an interstrand linkage to be formed between the docking strand and the nucleic acid barcode through the reactivity of the barcode's photo-reactive nucleobase, *e.g.,* CNVK base.

Data can also be written into a pattern or shape, *e.g.,* embedded in a hydrogel, such that the pattern or shape can encode information. In some embodiments, nucleic acids can be written so as to display a pattern or shape within another pattern or shape.

The nucleic acid pattern can be in the form of any pre-determined pattern engineered using methods provided herein. Alternatively, the nucleic acid pattern can be stochastic or random. Stated another way, nucleic acid patterns can be isotropic or anisotropic. Patterns can be in the form of lines, circles, tubes, spheres, fibers, letters, numbers, dots, polygons, squares, matrix barcode (*e.g.,* QR code), binary code, or any other pattern known in the art. The pattern can be any size, shape, or form that permits visualization or reading of the pattern.

A crosslinking strand can be designed to stably bind or transiently bind to a docking strand. In some embodiments, a crosslinking strand can be covalently or non-covalently bound to a docking strand. In some embodiments, a crosslinking strand can be complementary to a docking strand. A crosslinking strand can be at least 20%, at least 30%, at least 40%, at least 50%, at least 60%, at least 70%, at least 80%, or at least 90% complementary to a docking strand. Further, a crosslinking strand may bind to a complementary domain of a different nucleic acid barcode. In some embodiments, a crosslinking strand may bind to a complementary domain of a nucleic acid that comprises its own crosslinking strand but does not comprise a barcode strand.

An area of a specific size within a substrate or hydrogel, referred to as a 'pixel', may be utilized for writing and reading information. A pixel comprising many identical docking strands may be in one of two states - ON (bit value 1) or OFF (bit value 2). The bit content of a single pixel can further be increased by the use of multiple bit strands (FIG. 4B). In this case, strands are designed with complementary x/x* and y/y* such that during successive rounds of binding and washing they can form concatemer strands (FIG. 4C). This could be done, for example, by first introducing in the 1 bit strands corresponding to position (color) 0, crosslinking them to the appropriate pixels, washing off excess 1 bits, flowing in the 0 bit strands corresponding to that color, crosslinking, and so on until a concatemer with n bit strands crosslinked onto the docking strand has been created. In this way the bit content per pixel has been increased to n. Another way to increase the bit content per pixel is to embed multiple orthogonal sequences (colors) into the substrate or hydrogel, with different bit strands complementary to each of these colors (FIGs. 4D-4E). The procedure may be similar to that described for FIG. 4C, where bit strands corresponding to each color are introduced successively, with wash steps in between, except that no 0 bits are required.

Another way to increase the bit content per pixel is to embed multiple orthogonal sequences (colors) into the substrate or hydrogel, with different bit strands complementary to each of these colors (FIG. 4D-4E). By introducing multiple different possible combinations of orthogonal sequences (i.e. 'colors') per pixel, the information content per pixel can be increased dramatically compared to a simple 0 vs. 1, black vs. white encoding scheme. In this case, the procedure is similar to that described for FIG. 4C, where bit strands corresponding to each color are flowed in successively, with wash steps in between, except that no 0 bits are required. A final possibility is to utilize the same densely coated sequence domain (x*) onto which all barcode strands may bind (FIG. 4F). In this way, only one sequence needs to be embedded, but all possible barcode sequences can hybridize. By controlling the reaction kinetics so that only a small fraction of the sites are bound to the 1 bit barcode strand of interest at the time of cross-linking, the remaining sites are left available for future rounds of bit encoding by other 1 bit strands. For example, if 4 barcode strands are employed, then less than or equal to 1/4 of binding sites might be occupied at the time of cross-linking of the first bit, ensuring the remaining 3/4 are available for future binding.

Concatemer strands can be cleaved from the substrate or hydrogel and/or their corresponding docking strands such that they are suspended in solution with or dried with the substrate or hydrogel. This cleavage can occur by chemical methods, e.g., using an enzyme, or by physical methods. In some embodiments, cleavage of concatemer strands further increases the information density provided by these methods. In some embodiments, cleaved concatemer strands can be sequenced using known methods of nucleic acid sequencing, *e.g.,* nanopore-based sequencing.

An exemplary writing technology is based on the use of UV light to direct crosslinking of nucleic acids. Typical diagrams for the WRITE, READ, and RESET operations are depicted in FIG. 3. In the WRITE operation, a two-domain crosslinking strand (blue-pink) is first bound to a docking strand (blue). The crosslinking strand can comprise a CNVK base modification within its complementary domain, a sequence specific and photo-inducible nucleic acid crosslinking moiety, is utilized. CNVK has been demonstrated to crosslink both efficiently and quickly (>90% in ~1 second), thus facilitating rapid WRITE operations. After the base-paired complex is exposed to UV light at 365 nm wavelength, the crosslinking strand comprising a CNVK modification (in the complementary domain) forms an inter-strand covalent crosslink to the docking strand. Any crosslinking strand/docking strand complexes that are not exposed to UV light do not form a covalent crosslink. Crosslinked strands are robust against stringent washing steps (*e.g.,* low salt, high temperature, formamide), leaving only the crosslinked structures in place after a wash, and its attached complementary strand (pink) in a user-defined pattern. From an information theory perspective, the presence of a complementary strand after the washing step represents the writing of a single bit of information, wherein crosslinked complexes attached to the substrate or hydrogel represent a 1 (ON) bit, and bare docking strands attached to the substrate or hydrogel represent a 0 (OFF) bit.

The throughput of the light-directed method described in FIG. 3 can be greatly increased through the use of Digital Micromirror Devices (DMDs), which contain arrays of micromirrors that can be set to different angles to either allow (ON) or disallow (OFF) light to pass through. DMDs have been used to create projector displays, as well as to pattern synthesis of oligonucleotides on chips [13]. Using a DMD, specific pixels of a frame can be exposed to UV light (FIG. 5A), thus enabling pixel-specific crosslinking as described. By adjusting the focus of the projected light, the pixel size can also be tuned. For example, passing the light through an objective lens with higher magnification could enable 1 micron or smaller pixel feature size (diffraction limited). A DMD with X* Y pixels can be moved, or the sample itself can be moved, to enable many frames to be patterned rapidly (FIG. 5B). The writing may further be extended from 2D into 3D by using optical sectioning capabilities that are common in confocal microscopy. Adjusting the Z focus of the laser can specifically target and only crosslink the strands within a defined XYZ alignment, or voxel. Furthermore, this process can be multiplexed with spinning disc confocal microscopes to allow for several simultaneous lasers to illuminate the substrate or hydrogel.

In some embodiments, a substrate or hydrogel can be patterned using photo- and/or light-based patterning. In some embodiments, a substrate or hydrogel can be patterned through the use of photolithography masks, micromirrors, or lithography.

In some embodiments, the substrate or hydrogel is encoded with nucleic acids while in an expanded state. In some embodiments, the substrate or hydrogel is encoded with nucleic acids while in a compressed state.

### Encoding strategies

For data writing and/or patterning on a substrate (*e.g.,* glass surface), barcode concatemers generally will be copied and lyophilized to maximize stability and storage density. However, the spatial or positional information of each barcode will also be preserved in order to allow for later reconstruction of the data, *e.g.,* into a contiguous string of 0's and 1's or an arbitrary value, or a patterned barcode matrix (*e.g.,* QR barcode). To preserve the spatial information of the nucleic acid barcodes after copying, each barcoded concatemer will also carry a barcode sequence that encodes its original spatial position (FIG. 6). This is not to be construed as limiting as it is contemplated that the barcodes can comprise sensing functions in the presence of an analyte.

In some embodiments, data contained with an individual barcode strand can be encoded by a single base, *e.g.* 'A' = 0 and 'G' = 1. In some embodiments, multiple bases can encode the same bit identifier, *e.g.* 'A' and 'T' can both encode 0 while 'C' = 1 and 'G' can both encode 1. In some embodiments, bits can be encoded by a mixture of bases, *e.g.,* a 2:1 ratio of A:T at a particular position can encode 0 while a 3:1 ratio of A:T bases can encode 1. In some embodiments, bits can be encoded by a multiplicity of bases, *e.g.,* 'ACTTAC' can encode bit 0 and 'GCAGAT' can encode bit 1. In some embodiments, a multiplicity of bases can be 2, 3, 4, 5, 6, 7, 8, 9, 10, or at least 15 bases. This is helpful in the case of a sequence readout because barcode sequences are sufficiently different from each other so that even several errors by a polymerase could be still mapped back to the same barcode sequence. In some embodiments, data encoding is performed in bits (base 2 system). In other embodiments, data encoding is performed in trits (base 3 system), wherein three unique barcode sequences comprising optionally comprising 1 to 100 bases each would encode each of three trit identifiers.

In some embodiments, nucleic acid barcodes and/or patterned nucleic acids can be read using an imager, *e.g.,* a fluorescent imager. In the case of imaging-based data readout (see *e.g.* FIG. 4A), a barcode library of size n can encode 2^n possible combinations if presence of a barcode strand encodes a 1 and absence encodes a 0. This allows for n bits of data to be encoded in each pixel with at least 1, at least 2, at least 3, at least 4, at least 5, or at least 10 different sequences used (see FIG. 4B-4G). In some embodiments, the fraction of encoded strands (*e.g.,* fraction or percentage of 1 bases) also encodes information. In some embodiments, varying levels of imager strands can be quantified, *e.g.,* produce quantitative signals, as corresponding to different states. For example, if illumination of a barcode sequence A is controlled such that in some cases it is present at 33% occupancy and in other it is at 66% occupancy, then there are now four possible states of barcode A: 0%, 33%, 66%, and 100% instead of the original two possible states (0% or 100%). The number of bits encoded per barcode is thus doubled. The states can be read out through quantitative microscopy, which can determine the signal level and not just whether it is present or absent. For 'g' possible differential signal level states with 'n' different barcode sequences, there would now be n*g possible combinations and therefore log_2(n*g) bits could be stored per pixel.

In some embodiments, nucleic acid barcodes and/or patterned nucleic acids can be read using nucleic acid sequencing technologies, *e.g.,* sanger sequencing, deep sequencing, or nanopore-based sequencing.

As used herein, the term "nucleic acid sequencing" refers to a method of identifying individual nucleobases of a given nucleic acid. Methods of nucleic acid sequencing are known in the art such as cDNA and RNA sequencing, imaging-based methods such as NanoString and a wide range of methods that use PCR as well as qPCR. See, *e.g.,* Sanger, F. et. al., Proc. Natl. Acad. Sci. USA, 74:5463- 5467 1977; U.S. Patent Nos. 6,025,136 and 6,018,041, 7,473 ,767.

In some embodiments, the method further comprises amplifying a nucleic acid sequence. As used herein, the term "amplifying" refers to a step of submitting a nucleic acid sequence to conditions sufficient to allow for amplification of a polynucleotide if all of the components of the reaction are intact. Components of an amplification reaction include, *e.g.,* primers, a polynucleotide template, polymerase, nucleotides, and the like. The term "amplifying" typically refers to an "exponential" increase in target nucleic acid. However, "amplifying" as used herein can also refer to linear increases in the numbers of a select target sequence of nucleic acid, such as is obtained with cycle sequencing. Methods of amplifying and synthesizing nucleic acid sequences are known in the art. For example, see US Patent Nos. 7,906.282, 8,367,328, 5,518,900, 7,378,262, 5,476,774, and 6,638,722.

In some embodiments, amplifying the nucleic acid sequence comprises a polymerase chain reaction (PCR). PCR is well known to those of skill in the art; see, *e.g.,* U.S. Patent Nos. 4,683,195 and 4,683,202; and PCR Protocols: A Guide to Methods and Applications, Innis et al., eds, 1990. Exemplary PCR reaction conditions typically comprise either two or three step cycles. Two step cycles have a denaturation step followed by a hybridization/elongation step. Three step cycles comprise a denaturation step followed by a hybridization step followed by a separate elongation step.

In the case of sequencing-based readouts, the total number of possible coordinate positions that can be encoded with the barcode strategy will scale as BCn and the amount of data will scale as n × log(BC) where BC is the size of the barcode library and n is the number of concatemers. For example, consider a 2 barcode library (b0,b1) for encoding data, with each individual barcode assigned as bit 0 or bit 1. A separate barcode library can be utilized for address encoding. Given an 8 barcode library all coordinate positions for a typical DMD device can be encoded with 7 concatemers, providing 87 ~2e6 possible combinations. Thus each spatial coordinate can be assigned a unique barcode sequence identifier, which can then be read by sequencing. Multiple fields of DMD writing events can also be encoded with additional address barcodes. As demonstrated in FIG. 6, a total of 30 nucleic acid barcode concatemers per pixel can encode roughly 2.6 GB of data. Additional address concatemers will increase the amount of data stored exponentially. With 20 address barcodes and 20 data barcode concatemers, this storage strategy can store up to an exabyte of data. In some embodiments, a barcode library can be any conceivable size, *e.g.,* 2, 3, 4, 5, 6, 7, 8, 9, 10, at least 20, at least 25, or at least 50 barcode library. In some embodiments, there can exist any conceivable number of address barcodes, *e.g.,* 2, 3, 4, 5, 6, 7, 8, 9, 10, at least 20, at least 25, or at least 50 address barcodes.

In some embodiments, each pixel of a DMD will require a nucleic acid barcode comprised of 30-40 individual barcode strands, including both the barcode strand sequence identifier for the coordinate positions followed by the data itself. Copying the entire barcode sequence will produce a contiguous DNA strand that can be adapted for sequencing readout. Barcode combinations corresponding to unique spatial coordinates will be stitched together to reconstruct the bit order of the barcodes (e.g., DNA data barcodes).

A further extension of the encoding strategy uses combinations of barcodes that could be present at each possible concatemer position and de-convolution at the sequencing stage to identify the bit data encoded. For example, if illumination of a barcode sequence A using a DMD is controlled such that, in some cases the barcode is present at 33% occupancy and in other it is at 66% occupancy, then there are now four possible states of barcode A: 0%, 33%, 66%, and 100% instead of the original two possible states (0% or 100%). A similar strategy can be employed for all other barcodes and combinations of barcodes (*e.g.* a position might have 50% barcode A, 25% barcode B, and 25% barcode C). In this way, a strategy with BC possible barcode sequences and g possible frequency states excluding the empty state (i.e. 0% crosslinked, *e.g*. 20%, 40%, 60%, 80%, 100% would correspond to 5 states) could now encode log_2(BC multichoose g) bits per position (instead of the default of log_2(BC) bits per pixel. This is equivalent to log_2((BC + g - 1) choose g) bits per pixel. For example, given 10 barcode sequences and 5 unique states, the number of pixels that could be stored per pixel would be log_2(14 choose 5) = 10.97 bits per cycle per pixel, which would roughly equate to an order of magnitude higher in writing throughput and data density compared to a simple 0 vs. 1 writing scheme (FIG. 7). If sequences must be amplified before undergoing sequencing, unique molecular identifiers (UMIs) can be appended to primers to ensure that duplicate reads of individual concatemer sequences are excluded.

In addition, auxiliary sequences and sequences encoding polypeptides can be added to the barcodes. Non-limiting examples include PCR primers, surface-bound sequences, intermediate complementary binding domains, analyte binding domains, promoters, enhancers, repressors, labels, tags, or any other sequence known in the art.

In certain embodiments, the full length sequence for a single nucleic acid barcode is 20 bases long, wherein the barcode strand comprises 6 nucleotides and each of the two flanking alternating hybridization domains comprise 7 nucleotides. Assuming an average molecular weight of 330 daltons per base [4], this sums up to roughly 1 bit per 6600 daltons, assuming each barcode encodes for a single bit of information. This comes to roughly 1 bit in 1.09e-20 grams of DNA, or ~11 exabytes/gram of DNA.

The nucleic acid barcodes and/or docking strands provided herein can be stored in the form of any shape or pattern useful for a given application. A well-defined pattern of barcodes can be useful in the storage of special information (*e.g.,* for anti-counterfeit or molecular verification). For example, sequence specific patterns can be pre-determined and can be visualized or read in the presence of one or more molecular barcode-revealing agents. The nucleic acid barcodes provided herein can also be useful for combinatorial molecular verification systems, whereby individually addressable regions (*e.g.,* pixels) correspond to different barcodes. Multiple combinations of barcode strands can be used for stringent detection of nucleic acid sequences.

As used herein, a "barcode-revealing agent" refers to any substance, chemical constituent, chemical molecule of synthetic or biological origin that when exposed or in contact with the nucleic acids provided herein, permits reading and/or visualization of the nucleic acid barcodes. The barcode activating agent acts as a key or a passcode to a stored combination. The barcode activating agent can be an analyte, small molecule, phase-changing agent, salt, metabolite, compound, nucleic acid, polypeptide, or genomic editing system. The barcode-activating agent can be added to the substrate or compressible hydrogel or aqueous solution to induce crosslinking of the bit identifiers.

In some embodiments, the substrate comprising nucleic acid barcodes provided herein is exposed to a barcode-activating agent. In some embodiments, the substrate comprising nucleic acid barcodes provided herein is not exposed to a barcode-revealing agent.

By way of example only, the sensing function of the nucleic acids provided herein can be performed via hybridization of complementary or partially complementary nucleic acid strands or any analyte that can be tagged via nucleic acid strands. Non-limiting examples of nucleic acid tags that can be used in the this context include affinity probes (antibodies), proteins/peptides, nanoparticles, fluorophores, FRET components, spacers, Click Reaction Substrates, metallic labels, or any other moiety, modification, chemical spacers, proteins, nanobodies, aptamers, antigens, and chemical compositions capable of being detected with methods described herein (*e.g.,* light, fluorescence imaging, fluorescence microscopy, electron microscopy, atomic force microscopy, cryo-electron microscopy, cameras, electromagnetic sensors, the unaided human eye, mass spectrometry, Western Blots, gel electrophoresis, and other detectors). In certain embodiments, complementary strands can serve as barcode-revealing strands or agents, such that the encoded information is only revealed or capable of being sensed in the presence of said barcode-revealing strands or agents. These agents can be transiently or non-transiently bound to their complements, and in certain embodiments, multiple strands can bind cooperatively to a spatially tethered complementary strand. The patterned sensing can aid in highly multiplexed detection and/or positioning of analytes to be detected by secondary assays. Confirmation of the presence of an analyte can be in the form of a fluorescence or colorimetric readouts, or by any of the detection methods provided herein and known in the art.

In other embodiments of the methods provided herein, the signal from the patterns provided herein can be amplified to improve visibility or detectability of the barcodes. Exemplary amplification methods can include but are not limited to Tyramide Signal Amplification (TSA) and Rolling Circle Amplification (RCA). It is contemplated that TSA or RCA can be useful for multivalent sensing via creation of patterned barcoded surfaces that can sense and identify analytes of a desired composition. For example, the patterned surfaces can be used for the recognition of multiple domains in the same analyte or recognition of multiple components of a complex, such as a DNA nanostructure, or an oligonucleotide-tagged complex. Furthermore, the sensing function of the barcodes provided herein can be used for geometric sensing via creation of patterned barcoded substrates that can sense analytes of a desired molecular geometric organization.

In other embodiments, sensing can be accomplished by analytes that preferentially associate with or bind to nucleic acids, such as intercalating dyes (e.g., Sybr Green^{™}, Sybr Gold^{™}, EvaGreen^{™}, and Sybr Safe^{™}. In yet another embodiment of the methods provided herein, sensing of an analyte can be accomplished with electrochemical sensors, by eye, imaging technologies (e.g., cameras), and with a reading device such as a microscope. For example, any of the nucleic acid sensing mechanisms can serve as barcode-revealing systems, such that sensing strands, complexes, and compositions, conditionally reveal the pattern in the presence of the correct reagents. In the absence of the barcode-revealing agent, the pattern remains invisible. For example, in the presence of the correct nucleic acid strand or analyte, the pattern signal would become apparent. In certain embodiments, the reaction can be protected by controlling binding properties of strands such that any sequences that are not identical or substantially identical to the prescribed barcode-revealing agent or sequence would not be capable of revealing the pattern.

In some embodiments, where the pattern features are below the diffraction limit of light, e.g., patterns that have been compressed, the substrate can first be re-expanded before sensing or visualization. In other embodiments, the pattern can be used to direct sequences onto desired locations to create a platform for sensing of target nucleic acid strands based on physical proximity.

In some embodiments of the methods provided herein, barcode concatemers can be dissociated, cleaved, or removed from the substrate they were assembled on. In this context, part of the barcode sequence can contain information about positions or coordinates on the substrate. By way of example only, this would include the following: 1) where physically on the substrate that barcode sequences were assembled on the substrate or 2) where within a larger dataset the concatemer(s) encoded information belongs. In the latter case, this positional information can be used as an 'index' of where the data encoded in the concatemer is positioned in the context of a larger dataset. For example, bits of 0's and 1's belong in a larger string of data that has been split to be encoded amongst many concatemer barcodes. As an additional example, the index can contain position information about where the encoded string of bits belongs within the entirety of the full-length data string. Thus, in cases where concatemers have been removed from the substrate, the data encoded within them can typically be read via sequencing methods.

### Copying data and long term storage

In some embodiments, enzymes, *e.g.,* polymerases, can be used to copy the nucleic acid barcodes, *e.g.,* when barcode concatemers had been formed on a glass surface. Copied nucleic acid barcodes can be subsequently lyophilized and stored separately. Once lyophilized, DNA is stable and can achieve its maximum volumetric density, approaching exabytes/gram.

Nucleic acid barcodes can be stored for any reasonable amount of time. A reasonable amount of time can be measured in hours, days, weeks, months, years, or decades. In some embodiments, nucleic acid barcodes are stored for at least 1 hour, at least 6 hours, at least 12 hours, at least 18 hours, at least 24 hours, or at least 36 hours. In some embodiments, nucleic acid barcodes are stored for at least 1 day, at least 2 days, at least 3 days, at least 4 days, at least 5 days, at least 6 days, or at least 10 days. In some embodiments, nucleic acid barcodes are stored for 1 week, at least 2 weeks, at least 3 weeks, or at least 5 weeks. In some embodiments, nucleic acid barcodes are stored for at least 1 month, at least 2 months, at least 3 months, at least 6 months, at least 9 months, at least 15 months, or at least 18 months. In some embodiments, nucleic acid barcodes are stored for at least 1 year, at least 2 years, at least 3 years, at least 4 years, at least 5 years, at least 6 years, at least 7 years, at least 8 years, at least 9 years, or at least 10 years. In some embodiments, nucleic acid barcodes are stored for at least 1 decade, 2 decades, or at least 3 decades.

In some embodiments, all nucleic acid barcodes of a single sample, e.g., from the same substrate surface, are can be stored together. In other embodiments, nucleic acid barcodes of a single sample can be stored in separate locations. In some embodiments, nucleic acid barcodes are stored in a test tube, Eppendorf tube, or an equivalent. In some embodiments, nucleic acid barcodes are stored on filter paper, e.g., Whatman filter paper, via capillary action, and can be stored dried at room temperature, or transferred to a substrate, *e.g.* a compressible hydrogel, through surface to surface contacts.

Nucleic acid barcodes can be stored at room temperature, at low temperatures, or at high temperatures. In some embodiments, a low temperature is any temperature below 20 °C, 15 °C, 10 °C, 5 °C, 0 °C, -10 °C, -20 °C, -50 °C, or -78 °C. In some embodiments, room temperature is any temperature between 5-35 °C, 10-30 °C, 15-30 °C, 15-25 °C, 20-25 °C, about 23 °C, or about 20 °C.

Reading the data encoded by nucleic acid barcodes can be performed using commercial available sequencing platforms, including next generation sequencing, or nanopore sequencing. In some embodiments, to circumvent the crosslinked junctions of nucleic acid barcodes, each nucleic acid barcode can be hybridized and crosslinked, forming a long chain concatemer of the barcode sequence itself and the alternating hybridization domains. The gaps in the hybridized complex can be filled in with a gap-filling polymerase and ligated together to form one complete strand. Following gap-filling, the crosslinked strand can be reversed with illumination at 305 nm of light [11], creating a single-copy, contiguous DNA barcode sequence. The copied, contiguous barcode sequence can then be adapted for various other purposes, including sequencing, *e.g.,* by attaching a DNA barcode carrying a unique primer binding site that can serve as a site for polymerase copying (FIG. 8).

### Storing nucleic acid-encoded substrates

A nucleic acid-encoded substrate or hydrogel can be stored, *i.e.,* retained or kept, following the writing and/or reading steps. In some embodiments, a substrate or hydrogel is first compressed prior to storage. Compression of a substrate or hydrogel can occur by decreasing the concentration of water surrounding the substrate or hydrogel, by increasing the concentration of non-aqueous solvent (*e.g.,* organic solvents), by increasing the total ionic strength (*e.g.,* by increasing the salt concentration), by altering the temperature (*e.g.,* decreasing the temperature), by altering the electric or magnetic potential of the substrate or hydrogel, by altering the pressure upon the substrate or hydrogel (*e.g.,* increasing the pressure), changing the pH of the environment surrounding the substrate or hydrogel, and other feasible methods of compression.

In some embodiments, a substrate or hydrogel is compressed by removing water from the environment surrounding the substrate or hydrogel. In some embodiments, water is removed by evaporation. In some embodiments, at least 20% v:v, at least 40% v:v, at least 60% v:v, at least 80% v:v, or at least 95% v:v of the total volume of water is removed.

In some embodiments, a substrate or hydrogel is compressed by increasing the concentration of a non-aqueous solvent in the environment surrounding the substrate or hydrogel. A non-aqueous solvent may or may not be miscible with water. In some embodiments, a non-aqueous solvent is an organic solvent such as ethanol, methanol, isopropanol, acetonitrile, or hexanes. In some embodiments, the organic solvent is ethanol. In some embodiments, a hydrogel is compressed by a solution that contains at least 20% v:v, at least 40% v:v, at least 60% v:v, at least 80% v:v, at least 95% v:v, or 100% v:v non-aqueous solvent.

In some embodiments, a substrate or hydrogel is compressed by increasing the total ionic concentration in the environment surrounding the substrate or hydrogel. In some embodiments, the total ionic concentration is increased by increasing the concentration of solutes. In some embodiments, a solute is a salt such as sodium chloride, potassium chloride, or a phosphate. In some embodiments, a hydrogel is compressed by increasing the solute concentration to at least 5% w:v, at least 10% w:v at least 20% w:v, at least 40% w:v, at least 60% w:v, at least 80% w:v, or at least 95% w:v.

In some embodiments, a substrate or hydrogel can be dried or desiccated. In some embodiments, a substrate or hydrogel is dried or desiccated using a vacuum desiccator, by removal of water, or by increasing the concentration of non-aqueous solvent. In some embodiments, the methods of compressing a substrate or hydrogel are also capable or drying or desiccating the substrate or hydrogel.

A substrate or compressed hydrogel can be stored for any reasonable amount of time. A reasonable amount of time can be measured in hours, days, weeks, months, years, or decades. In some embodiments, a substrate or hydrogel is stored for at least 1 hour, at least 6 hours, at least 12 hours, at least 18 hours, at least 24 hours, or at least 36 hours. In some embodiments, a substrate or hydrogel is stored for at least 1 day, at least 2 days, at least 3 days, at least 4 days, at least 5 days, at least 6 days, or at least 10 days. In some embodiments, a substrate or hydrogel is stored for 1 week, at least 2 weeks, at least 3 weeks, or at least 5 weeks. In some embodiments, a substrate or hydrogel is stored for at least 1 month, at least 2 months, at least 3 months, at least 6 months, at least 9 months, at least 15 months, or at least 18 months. In some embodiments, a substrate or hydrogel is stored for at least 1 year, at least 2 years, at least 3 years, at least 4 years, at least 5 years, at least 6 years, at least 7 years, at least 8 years, at least 9 years, or at least 10 years. In some embodiments, a substrate or hydrogel is stored for at least 1 decade, 2 decades, or at least 3 decades.

A substrate or hydrogel can be stored in an aqueous solution (*e.g.,* water-based), a non-aqueous solution (*e.g.* organic solvent, *e.g.,* ethanol), or completely dry (*i.e.* no present solution or solvent). In some embodiments, a substrate or hydrogel can be stored alone or with other substrates or hydrogels. In some embodiments, the entirety of a substrate or hydrogel can be stored together. In other embodiments, portions of a substrate or hydrogel or its components can be stored in separate locations. In some embodiments, a substrate or hydrogel is stored on a flat surface. In some embodiments, a substrate or hydrogel is stored in a test tube, Eppendorf tube, or an equivalent. In some embodiments, a substrate or hydrogel is stored on filter paper, *e.g.,* Whatman filter paper, via capillary action, and can be stored dried at room temperature.

A substrate or hydrogel can be stored at room temperature, at low temperatures, or at high temperatures. In some embodiments, a low temperature is any temperature below 20 °C, 15 °C, 10 °C, 5 °C, 0 °C, -10 °C, -20 °C, -50 °C, or -78 °C. In some embodiments, room temperature is any temperature between 5-35 °C, 10-30 °C, 15-30 °C, 15-25 °C, 20-25 °C, about 23 °C, or about 20 °C.

### Reading nucleic acid-encoded substrates

In some embodiments, a plurality of nucleic acids is capable of binding to the nucleic acid-encoded pattern and/or nucleic acid barcodes embedded within a substrate or compressible hydrogel. In some embodiments, the plurality of nucleic acids can further comprise a detectable moiety, *e.g.,* a fluorescent molecule. In these embodiments, binding of the plurality of nucleic acids comprising a detectable moiety to the nucleic acid-encoded pattern and/or nucleic acid barcodes enables detection, *i.e.,* reading, of the information encoded by the pattern and/or barcodes.

In some embodiments, as described above, a nucleic acid that is capable of binding to a nucleic acid-encoded pattern further comprises a detectable moiety. In some embodiments, the detectable moiety is detectable following exposure of the barcodes provided herein to a barcode-activating agent. In some embodiments, the detectable moiety is detectable without exposure to a barcode-activating agent.

As used herein a "detectable moiety" or "label" refers to a molecular entity that is capable of being detected, *e.g.,* a fluorophore, a colorimetric dye, a pigment, an optically-active agent. Detectable moieties can be covalently linked or non-covalently linked to a nucleic acid. Detectable moieties can be visualized using the naked, unaided eye, a microscope, a light sheet microscope, a fluorescent scanner, a spectrophotometric scanner, an electrical voltammeter, or any other detection method. In some embodiments, a detectable moiety is a fluorophore, *e.g.,* an organic fluorophore or an inorganic fluorophore. In some embodiments, a detectable moiety is not fluorescent. In some embodiments, a detectable moiety is not a fluorophore. The detectable moiety can be detected using any method known in the art. Non-limiting examples of detection methods include, photoimaging, light microscopy, fluorescence microscopy, atomic force microscopy, Förster resonance energy transfer (FRET), and spectrometry.

In some embodiments, a detectable moiety is not bound to a nucleic acid. In some embodiments, a detectable moiety that is not bound to a nucleic acid can bind directly to a nucleic acid-encoded pattern. In some embodiments, a detectable moiety can bind covalently or non-covalently to a nucleic acid-encoded pattern. In some embodiments, a detectable moiety can specifically bind to a nucleic acid. In some embodiments, a detectable moiety can be Sybr Gold^{™}, Sybr Green^{™}, Sybr Safe^{™} DAPI, Hoehst or another dye that binds non-specifically to nucleic acids, such as by intercalating into base pairs.

Fluorophores are chemical or molecular entities that absorb electromagnetic energy of certain characteristic wavelengths and thereafter emit electromagnetic energy at other characteristic wavelengths. Organic fluorophores, which include molecular dyes, fluorescent proteins, and intrinsic fluorophores, typically comprise aromatic groups, planar or cyclic molecules with several π bonds. Inorganic fluorophores, which include lanthanides and quantum dots, typically comprise metals and/or nanoparticles. In the methods provided herein, any classification or family of fluorophores can be utilized. Exemplary fluorophores for use in the methods described can include Alexa Fluor 488 (AF488), Alexa Fluor 647 (AF647), Texas Red, fluorescein, rhodamine, coumarin, cyanine, Oregon Green, other Alexa Fluor dyes, eosin, dansyl, prodan, anthracenes, anthtraquinones, cascade blue, Nile Red, Nile Blue, cresyl violet, acridine orange, acridine yellow, crysal violet, malachite green, BODIPY, Atto, Tracy, Sulfo Cy dyes, HiLyte Fluor, and derivatives of each thereof. Further non-limiting examples of useful fluorophores are known in the art (see, *e.g*. Stockert, J.C and Blázquez-Castro, A. Chapter 3 Dyes and Fluorochromes, Fluorescence Microscopy in Life Sciences. 2017, Bentham Science Publishers. pp. 61-95.; Herman B. Absorption and emission maxima for common fluorophores, Curr. Protoc. Cell Biol. 2001, Appendix 1:Appendix 1E.).

In some embodiments, a detectable moiety can be a protein, *e.g.,* a fluorescent protein or an enzyme. In some embodiments, a fluorescent protein for use as a detectable moiety can be a green fluorescent protein (GFP), a cyan fluorescent protein, or a yellow fluorescent protein. A fluorescent protein can be as described in Day, R.N. and Davidson, M.W. The fluorescent protein palette: tools for cellular imaging. Chem Soc Rev. 2009 Oct; 38(10): 2887-2921. In some embodiments, an enzyme for use as a detectable moiety can be a peroxidase enzyme, *e.g.,* horseradish peroxidase. In some embodiments, a peroxidase enzyme enables visualization of the nucleic acid-encoded pattern through its enzymatic activity to oxidize a peroxidase substrate. Exemplary peroxidase substrates include 3'-Diaminobenzidine (DAB); 3,3',5,5'-Tetramethylbenzidine (TMB); 2,2'-azino-bis(3-ethylbenzothiazoline-6-sulphonic acid) (ABTS); luminol; homovanillic acid; o-Phenylenediamine (OPD); AmplexRed; and 3-Amino-9-ethylcarbazole (AEC).

In some embodiments, a detectable moiety is a nanoparticle, *e.g.,* a heavy metal, *e.g.,* a gold nanoparticle. In some embodiments, a nanoparticle can be visualized using microscopy, *e.g.,* light microscopy or atomic force microscopy.

To read the data pattern, the presence of the complementary (pink) strand can be probed (READ, FIG. 3). This can be done, for example, with a nucleic acid comprising a detectable moiety (*e.g.,* a fluorescent molecule) that binds (*e.g.,* is complementary) to the complementary strand. The nucleic acid comprising a detectable moiety would only bind to the ON bits (*i.e.,* strands that contain a complementary strand), and then the pattern could be read, *e.g.,* fluorescence on a microscope or other fluorescent scanner.

Resetting the substrate or hydrogel (RESET) so that it can return to its original written state typically requires a wash step that removes the nucleic acid(s) comprising a detectable moiety and re-exposes any complementary strands. In some embodiments, the substrate or gel is reset by removing the plurality of nucleic acids that comprise a detectable moiety. In some embodiments, the plurality of nucleic acids that comprise a detectable moiety are removed temporarily by disrupting the binding interaction between the plurality of nucleic acids and the nucleic acid-encoded pattern of the substrate or hydrogel. In some embodiments, new data can be written by appending a new plurality of nucleic acid barcodes to a concatemer, with the expectation that the newer barcodes take precedence over earlier written barcodes in the concatemer. In some embodiments, a substrate (*e.g.,* a hydrogel) can be compressed and optionally stored following a resetting of the substrate or hydrogel.

In some embodiments, the data within the concatemer sequence are copied, and the copy is read through nucleic sequencing as provided herein. In some embodiments, the concatemers themselves can be directly read. For concatemers of alternating strand information with single-stranded barcode gaps, the gaps can be filled with a polymerase enzyme before the strands are ligated to create an identifiable barcode record (*e.g.,* identified by next generation sequencing methods; see also **FIG. 8**).

### Re-writing nucleic acid-encoded substrates

The nucleic acid-encoded pattern of a substrate or hydrogel can be permanently removed or "erased" from a substrate or hydrogel at any step, leaving behind only the docking strands embedded within the substrate or hydrogel. In some embodiments, the nucleic acid-encoded pattern is permanently removed from the substrate or hydrogel following a READ step.

In some embodiments, a photo-induced crosslink can be reversed, thereby permanently removing the pattern. In some embodiments, a CNVK crosslink can be reversed when exposed to 305 nm UV light, removing the crosslinked strand, *e.g.,* erasing the data that was previously written in. In some embodiments, the re-exposed docking strand can be reused to hybridize a new crosslinking strand carrying the same or different complementary strand. In some embodiments, the re-exposed docking strand can be hybridized to a new set of barcoded concatemer strands, thereby re-writing the data content for that docking strand. In some embodiments, a new nucleic acid-encoded pattern can be designed using a different lithography or DMD method than had previously been used. In some embodiments, multiple pixels of data can be erased and re-written simultaneously, *e.g.,* by using a DMD.

In some embodiments, the nucleic acid-encoded pattern can be removed using enzymatic methods. In some embodiments, an enzyme can be used to reverse the crosslink between a docking strand and a crosslinking strand.

### Further embodiments

In some embodiments, nucleic acid barcodes can be functionalized and/or patterned onto any reasonable substitute for a substrate or compressible hydrogel that is capable of being physically or chemically compressed for the purposes of increased data storage density. In some embodiments, a reasonable substitute for a substrate or compressible hydrogel can be any shrinkable material, including but not limited to heat-shrink plastic and viscoelastic foam (memory foam).

In some embodiments, the primary preparation step, *i.e.,* functionalization, required for a substrate surface to be compatible with light directed nucleic acid data writing is functionalization of the surface with nucleic acid docking strands, as provided herein. In some embodiments, functionalization can be performed through direct incorporation of the dockings strands into a polymer network, *e.g.,* a compressible hydrogel, using a modified nucleic acid that comprises at least one monomeric unit. In some embodiments, functionalization can be performed through substrate surface treatment using nonspecific interactions of nucleic acids with the substrate.

As provided herein, nucleic acid barcodes can be detected by the presence of one bound fluorophore (encode 1) or absence of a fluorophore (encode 0). Further, in some embodiments, more potential states can be introduced, *e.g.,* presence of two bound fluorophores (encode 2). For example, two bound fluorophores will be interpreted as double the fluorescent signal when compared to one bound fluorophore. In some embodiments, the method for light directed nucleic acid data writing can be modified such that each nucleic acid can be conjugated to a fluorescent material of interest and spatially addressed onto the substrate surface, *e.g*. glass or compressible hydrogels, via a DMD and photo crosslinking. In these embodiments, data can be read using a high speed camera recording the position of each fluorophore, *e.g.,* single molecule fluorescent readings.

In some embodiments, nucleic acid-encoded patterns and/or barcodes can be read or visualized using colorimetric reactions, *e.g.,* 3'-Diaminobenzidine (DAB) oxidation or horseradish peroxidase (HRP) enzymatic activity. In certain embodiments, reading or visualization using colorimetric reactions can be induced to take place in a restricted spatial position.

In some embodiments, a substrate or compressible hydrogel can be patterned with nucleic acids, optionally nucleic acid barcodes, using any physical or chemical methods, or by photo patterning with the use of masks and lithography. DMD can be classified as 'maskless' lithography due to the fine spatial control afforded by the >1e6 micromirrors within the DMD array. In some embodiments, the same spatial illumination profile as can be provided by a DMD can be achieved with traditional 'masked' illumination wherein the illumination source is a single point source, and wherein a pre-cut mask is placed in front of the light source to create a spatial illumination profile.

In some embodiments, substrate or hydrogels including compressible hydrogels can be pre- or post- casted in gels or other embedding materials which can optionally comprise varying properties, e.g., differential sensitivity to ionic strength or temperature. For example, hydrogels can be molded or fitted into specific shapes by polymerizing an acrylamide solution directly inside of a preset mold.

In some embodiments, the flow of components, e.g., buffers or nucleic acid barcodes, through a compressible hydrogel can be facilitated by external stimuli, e.g., electric field, magnetic field, pressure, suction, capillary action, or dehydration. In some embodiments, the use of electric can expedite the transportation of nucleic acids, e.g., nucleic acid barcodes or docking strands, into the hydrogel.

In some embodiments, nucleic acid barcodes will be removed or cleaved from a substrate surface to which they are bound. Cleavage protocols will vary depending on the substrate surface used. For example, cleavage of strands that are non-covalently bound to a surface, *e.g.,* a glass surface, will generally require mild conditions, *e.g.,* gentle heat and/or formamide. In some embodiments, cleavage from a compressible surface, *e.g.,* a hydrogel or a shrinkable plastic, can use mild denaturants, *e.g.,* guanidinium chloride, to denature the compressible surface. In some embodiments, a mild denaturant can degrade the substrate, leaving only the strands in solution.

In some embodiments, the docking strand bound to a substrate surface can comprise a homology domain for an endonuclease, such that concatemer constructs become cleaved from the surface-bound docking strands upon introduction of an endonuclease enzyme. Some embodiments include a deoxy-Uracil base in a docking sequence, such that it can be cleaved upon introduction of a USER enzyme. Some embodiments utilize RNA bases in the docking sequence, such that an RNAse H family enzyme can be used to digest the RNA and cleave the concatemer construct from the surface.

### EXAMPLES

### Example 1: Patterning of nucleic acids on compressible hydrogel

Nucleic acid patterning was experimentally validated on a compressible hydrogel, as depicted in FIGs. 6A-6D. A compressible hydrogel was first embedded with docking strands using acrydite chemistry during polymerization of the hydrogel matrix. The hydrogel comprising acrylamide monomers, TEMPO, TEMED and 2-4 µM acrydite modified DNA (docking strand) was polymerized at room temperature after mixing all components. Fluorophore-labeled CNVK-modified crosslinking strands (sequence 'a' in FIG. 10A) were then prepared at a concentration of 0.5 µM in phosphate buffered saline and incubated overnight with the hydrogel. Sequence 'a'is TCGAXGCAT, wherein X is CNVK. After the hydrogel was incubated with CNVK imager strands, a DMD with a 405 nm LED was used to generate a checkerboard pattern onto the hydrogel surface for one minute (FIG. 10B) (WRITE). After washing to remove non-crosslinked strands, the gel was then imaged (READ) on a fluorescent scanner. The hydrogel was then physically compressed by incubating in 100% ethanol overnight (FIG. 10C) before imaging on the fluorescent scanner. Comparative images to show the scale of the patterned hydrogel before and after physical compression can be seen in FIG. 10D.

Several additional sequences were subsequently validated for use in photopatterning experiments, as done above for Sequence 'a' (TCGAXGCAT). For each sequence 1-6 provided below, X represents a photoreactive crosslinking base modification (CNVK).
1. GCATACCTCCTAATTCCC TTCA CCTATCTCTA X CTCCAGC (SEQ ID NO: 1)
2. GGGAATTA X GAGGTATGC GCTC GCTGGAGATAGAGATAGG (SEQ ID NO: 2)

TCGA X GCAT ccc ATGATGT TGTGGT GTTTATT AATAAAC ggg GTTG T
TGTG (SEQ ID NO: 3)

CACA X CAAC ccc GTTTATT TGTGGT ATGATGT ACATCAT ggg ATGC T
TCGA (SEQ ID NO: 4)

TCGA X GCAT ccc ATGATGT AATAAG GTTTATT AATAAAC ggg GTTG T
TGTG (SEQ ID NO: 5)

CACA X CAAC ccc GTTTATT AATAAG ATGATGT ACATCAT ggg ATGC T
TCGA (SEQ ID NO: 6)

### Example 2: Concatemerization of DNA to generate pattern

Two DNA barcodes (Sequences 3 and 4 of the sequences provided in Example 1) were utilized to generate a series of nucleic acid barcode concatemers. We have performed initial concatemerization experiments using a DLP9500 DMD from Texas Instruments to spell out the letters 'MIST' onto a slide with iterative concatemeriztion. Each hybridization event was performed using 1x Phosphate Buffered Saline (PBS) for a duration of 1 minute, followed by a 10 second illumination at the 365 nm wavelength for UV crosslinking. Excess strands were washed away in 0.05x PBS buffer to complete one cycle of hybridization and crosslinking. The next iteration of hybridization and crosslinking was repeated with another DNA barcode to generate a nucleic acid barcode concatemer. The experiment is designed such that each letter in 'MIST' will contain iteratively more fluorescently labeled barcoded sequences, with the expectation of the weakest fluorescent signal for the letter 'M' followed by progressively stronger signals up to the highest at the letter 'T'. In FIG. 11, we can see that the results roughly follow this trend (Note that some light was cut off by the objective, leading to uneven illumination at the edges. The final letter 'T' contained four DNA barcode sequences with an alternating 0-1-0-1-bit encoding scheme.

### References

[1] Bancroft, C., Bowler, T., Bloom, B. & Clelland, C. T. Long-term storage of information in DNA. Science 293, 1763-1765 (2001).
[2] Church, G. M., Gao, Y. & Kosuri, S. Next-generation digital information storage in DNA. Science 337, 1628 (2012).
[3] Ionov, L. Hydrogel-based actuators: possibilities and limitations. Mater. Today 17, 494-503 (2014).
[4] Bahram, M., Mohseni, N. & Moghtader, M. An Introduction to Hydrogels and Some Recent Applications. in Emerging Concepts in Analysis and Applications of Hydrogels (ed. Majee, S. B.) (InTech, 2016).
[5] Cangialosi, A. et al. DNA sequence-directed shape change of photopatterned hydrogels via high-degree swelling. Science 357, 1126-1130 (2017).
[6] Sun, J.-Y. et al. Highly stretchable and tough hydrogels. Nature 489, 133-136 (2012).
[7] Chen, F., Tillberg, P. W. & Boyden, E. S. Optical imaging. Expansion microscopy. Science 347, 543-548 (2015).
[8] Chang, J.-B. et al. Iterative expansion microscopy. Nat. Methods 14, 593-599 (2017).
[9] Truckenbrodt, S. et al. X10 expansion microscopy enables 25-nm resolution on conventional microscopes. EMBO Rep. e45836 (2018).
[10] Yoshimura, Y. & Fujimoto, K. Ultrafast reversible photo-cross-linking reaction: toward in situ DNA manipulation. Org. Lett. 10, 3227-3230 (2008).
[11] Vieregg, J. R., Nelson, H. M., Stoltz, B. M. & Pierce, N. A. Selective nucleic acid capture with shielded covalent probes. J. Am. Chem. Soc. 135, 9691-9699 (2013).
[12] Erlich, Y. & Zielinski, D. DNA Fountain enables a robust and efficient storage architecture. Science 355, 950-954 (2017).
[13] Dudley, D., Duncan, W. M. & Slaughter, J. Emerging digital micromirror device (DMD) applications. in MOEMS Display and Imaging Systems 4985, 14-26 (International Society for Optics and Photonics, 2003).
[14] Li, J., Czajkowsky, D. M., Li, X. & Shao, Z. Fast immuno-labeling by electrophoretically driven infiltration for intact tissue imaging. Sci. Rep. 5, 10640 (2015).

### Example 3: Additional Embodiments of Concatemerization of DNA to Generate Patterns

Surface patterning of the nucleic acid barcodes were validated on a glass slide functionalized with a uniform layer of docking strands.

Docking strands comprised a hybridization sequence, a crosslinking strand, and a biotin functional group. Glass surfaces were passivated with BSA-biotin and streptavidin to serve as a binding substrate for the biotinylated docking strand. Crosslinking strands were substantially identical to the sequence 'a' in FIG 10A, comprising a CNVK photoreactive nucleotide and a Cy3b fluorophore. Crosslinking strands were hybridized to the docking strand at 100 nM concentration in 1x PBS buffer with 500 mM NaCl.

A DMD photomask was used to generate the patterns in FIG. 12 and FIG. 13. FIG. 12 utilized a photomask whereby only a single mirror on a DMD array was flipped to an 'on' position at regularly spaced intervals.

FIG. 13 utilized a photomask generated from a photograph. A 365 nm LED light source was used as the UV light source to initiate crosslinking. The UV illumination profile of the DMD was then focused through a 10x microscope objective onto the glass surface containing the docking and crosslinking strands. Crosslinking was performed and completed within a 1 second UV illumination time. Excess un-crosslinked strands were washed away in 1x PBS buffer. The spatial profile of the crosslinking was verified by a tile scan in the TRITC channel with a 40x objective and determined the correct pattern.

The indefinite articles "a" and "an," as used herein in the specification and in the claims, unless clearly indicated to the contrary, should be understood to mean "at least one." The singular terms "a," "an," and "the" include plural referents unless context clearly indicates otherwise. Similarly, the word "or" is intended to include "and" unless the context clearly indicates otherwise. The abbreviation, "*e.g.*" is derived from the Latin *exempli gratia,* and is used herein to indicate a non-limiting example. Thus, the abbreviation "*e.g*." is synonymous with the term "for example."

The term "substantially identical" means two or more nucleotide sequences have at least 50%, 60%, 65%, 70%, 80%, 85%, 90%, 95%, or 97% identical nucleotides. In some embodiments, "substantially identical" means two or more nucleotide sequences have the same identical nucleotides.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Stated another way, the terms "comprising" or "comprises" are used in reference to compositions, methods, and respective component(s) thereof, that are essential to the method or composition, yet open to the inclusion of unspecified elements, whether essential or not.

Only the transitional phrases "consisting of" and "consisting essentially of" shall be closed or semi-closed transitional phrases, respectively, as set forth in the United States Patent Office Manual of Patent Examining Procedures, Section 2111.03. The term permits the presence of additional elements that do not materially affect the basic and novel or functional characteristic(s) of that embodiment of the invention.

The term "statistically significant" or "significantly" refers to statistical significance and generally means a two standard deviation (2SD) or greater difference.

The terms "about" and "substantially" preceding a numerical value mean ±10% of the recited numerical value.

Where a range of values is provided, each value between the upper and lower ends of the range are specifically contemplated and provided herein.

## Claims

1. A method of writing or storing data, comprising:
photocrosslinking a first nucleic acid comprising a barcode domain flanked by a pair of hybridization domains to a second nucleic acid comprising a barcode domain flanked by a pair of hybridization domains;
wherein at least one of the hybridization domains of each pair comprises a photoreactive element; and
producing a concatemer of barcodes.

2. The method of claim 1, further comprising photocrosslinking to the first or second nucleic acid at least one additional nucleic acid that comprises a barcode domain flanked by a pair of hybridization domains.

3. The method of claim 1 or claim 2, wherein each barcode domain is assigned an independent bit value.

4. The method of any preceding claim, wherein the photoreactive element is:
(a) a photoreactive nucleotide; or
(b) psoralen.

5. The method of claim 4, wherein the photoreactive nucleotide is a CNVK or CNVD crosslinking base.

6. The method of any preceding claim, wherein:
(a) the photocrosslinking is performed using a 350-400 nm, optionally a 365 nm, wavelength of light; and/or
(b) the photocrosslinking is performed in aqueous solution; and/or
(c) the method is enzyme-free.

7. The method of any one of claims 1-6, wherein a barcode of the first and/or second nucleic acid is selected from a barcode library having a minimum Hamming distance of 4.

8. The method of any one of claims 1-7, wherein the first and/or second nucleic acid is attached to a substrate, optionally wherein the first and/or second nucleic acid is attached to the substrate in a predetermined pattern.

9. The method of claim 8, wherein the substrate is selected from the group consisting of: glass, transparent polymers, polystyrene, hydrogels, metal, ceramic, paper, agarose, gelatin, alginate, dextran, iron oxide, stainless steel, gold, copper, silver chloride, polycarbonate, polydimethylsiloxane, polyethylene, acrylonitrile butadiene styrene, cyclo-olefin polymers, cyclo-olefin copolymers, streptavidin, resin, and a biological material.

10. The method of claim 8, wherein the substrate is a compressible hydrogel.

11. The method of claim 9, wherein the biological material is selected from the group consisting of: a tissue, a cell, an organoid, an engineered tissue; and an extracellular matrix.

12. The method of any one of claims 1-11, wherein the concatemer of barcodes encode special information and/or spatial information, optionally, wherein the special information is selected from the group consisting of: text, images, coordinates, graphics, movies, sequencing data, QR codes, binary codes, and health records.

13. The method of any one of claims 1-12, wherein each of the nucleic acids is covalently linked to at least one other of the nucleic acids through a single photoreactive element of a hybridization domain.

14. The method of any one of claims 1-13, wherein each barcode has a length of at least 5 nucleotides, optionally 5-10 nucleotides.

15. The method of any one of claims 1-14, wherein the nucleic acid barcodes can be read using:
(a) nucleic acid sequencing technologies; or
(b) a fluorescence or colorimetric readout.

## Patentansprüche

1. Verfahren zum Schreiben oder Speichern von Daten, umfassend:
Photokreuzvernetzen einer ersten Nukleinsäure, die eine Barcode-Domäne umfasst, die von einem Paar Hybridisierungsdomänen flankiert ist, mit einer zweiten Nukleinsäure, die eine Barcode-Domäne umfasst, die von einem Paar Hybridisierungsdomänen flankiert ist;
wobei mindestens eine der Hybridisierungsdomänen jedes Paares ein photoreaktives Element umfasst; und
Erzeugen eines Concatemers von Barcodes.

2. Verfahren nach Anspruch 1, ferner umfassend Photokreuzvernetzen der ersten oder der zweiten Nukleinsäure mit mindestens einer weiteren Nukleinsäure, die eine Barcode-Domäne umfasst, die von einem Paar Hybridisierungsdomänen flankiert ist.

3. Verfahren nach Anspruch 1 oder Anspruch 2, wobei jeder Barcode-Domäne ein unabhängiger Bitwert zugewiesen ist.

4. Verfahren nach einem vorhergehenden Anspruch, wobei das photoreaktive Element Folgendes ist:
(a) ein photoreaktives Nukleotid; oder
(b) Psoralen.

5. Verfahren nach Anspruch 4, wobei das photoreaktive Nukleotid eine CNVK- oder CNVD-Kreuzvernetzungsbase ist.

6. Verfahren nach einem vorhergehenden Anspruch, wobei:
(a) das Photokreuzvernetzen unter Verwendung einer Lichtwellenlänge von 350-400 nm, optional 365 nm, durchgeführt wird; und/oder
(b) das Photokreuzvernetzen in wässriger Lösung durchgeführt wird; und/oder
(c) das Verfahren enzymfrei ist.

7. Verfahren nach einem der Ansprüche 1 bis 6, wobei ein Barcode der ersten und/oder der zweiten Nukleinsäure aus einer Barcode-Bibliothek mit einem minimalen Hamming-Abstand von 4 ausgewählt wird.

8. Verfahren nach einem der Ansprüche 1 bis 7, wobei die erste und/oder die zweite Nukleinsäure an ein Substrat gebunden wird, wobei gegebenenfalls die erste und/oder die zweite Nukleinsäure in einem vorbestimmten Muster an das Substrat gebunden wird.

9. Verfahren nach Anspruch 8, wobei das Substrat ausgewählt wird aus der Gruppe bestehend aus: Glas, transparenten Polymeren, Polystyrol, Hydrogelen, Metall, Keramik, Papier, Agarose, Gelatine, Alginat, Dextran, Eisenoxid, Edelstahl, Gold, Kupfer, Silberchlorid, Polycarbonat, Polydimethylsiloxan, Polyethylen, Acrylnitril-Butadien-Styrol, Cycloolefin-Polymeren, Cycloolefin-Copolymeren, Streptavidin, Harz und einem biologischen Material.

10. Verfahren nach Anspruch 8, wobei das Substrat ein komprimierbares Hydrogel ist.

11. Verfahren nach Anspruch 9, wobei das biologische Material ausgewählt wird aus der Gruppe bestehend aus: einem Gewebe, einer Zelle, einem Organoid, einem künstlichen Gewebe und einer extrazellulären Matrix.

12. Verfahren nach einem der Ansprüche 1 bis 11, wobei das Concatemer von Barcodes spezielle Informationen und/oder räumliche Informationen kodiert, wobei die speziellen Informationen optional ausgewählt werden aus der Gruppe bestehend aus: Text, Bildern, Koordinaten, Grafiken, Filmen, Sequenzdaten, QR-Codes, Binärcodes und Gesundheitsdatensätzen.

13. Verfahren nach einem der Ansprüche 1 bis 12, wobei jede der Nukleinsäuren über ein einzelnes photoreaktives Element einer Hybridisierungsdomäne kovalent mit mindestens einer anderen Nukleinsäure verbunden ist.

14. Verfahren nach einem der Ansprüche 1 bis 13, wobei jeder Barcode eine Länge von mindestens 5 Nukleotiden, optional 5 bis 10 Nukleotiden aufweist.

15. Verfahren nach einem der Ansprüche 1 bis 14, wobei die Nukleinsäure-Barcodes gelesen werden können unter Verwendung von:
(a) Nukleinsäuresequenzierungstechnologien; oder
(b) einer Fluoreszenz- oder kolorimetrischen Messung.

## Revendications

1. Procédé d'écriture ou de stockage de données, comprenant :
la photoréticulation d'un premier acide nucléique comprenant un domaine de code-barres flanqué d'une paire de domaines d'hybridation à un second acide nucléique comprenant un domaine de code-barres flanqué d'une paire de domaines d'hybridation ;
dans lequel au moins un des domaines d'hybridation de chaque paire comprend un élément photoréactif ; et
la production d'un concaténeur de codes-barres.

2. Procédé selon la revendication 1, comprenant également la photoréticulation au premier ou au second acide nucléique d'au moins un acide nucléique supplémentaire qui comprend un domaine de code-barres flanqué d'une paire de domaines d'hybridation.

3. Procédé selon la revendication 1 ou la revendication 2, dans lequel chaque domaine de code-barres se voit attribuer une valeur binaire indépendante.

4. Procédé selon une quelconque revendication précédente, dans lequel l'élément photoréactif est :
(a) un nucléotide photoréactif ; ou
(b) un psoralène.

5. Procédé selon la revendication 4, dans lequel le nucléotide photoréactif est une base de réticulation de CNVK ou de CNVD.

6. Procédé selon une quelconque revendication précédente dans lequel :
(a) la photoréticulation est réalisée à l'aide d'une longueur d'onde de lumière de 350 à 400 nm, éventuellement de 365 nm ; et/ou
(b) la photoréticulation est réalisée en solution aqueuse ; et/ou
(c) le procédé est sans enzyme.

7. Procédé selon l'une quelconque des revendications 1 à 6, dans lequel un code-barres du premier et/ou du second acide nucléique est sélectionné à partir d'une bibliothèque de codes-barres ayant une distance de Hamming minimale de 4.

8. Procédé selon l'une quelconque des revendications 1 à 7, dans lequel le premier et/ou le second acide nucléique est fixé à un substrat, éventuellement dans lequel le premier et/ou le second acide nucléique est fixé au substrat selon un motif prédéterminé.

9. Procédé selon la revendication 8, dans lequel le substrat est choisi dans le groupe constitué de : verre, polymères transparents, polystyrène, hydrogels, métal, céramique, papier, agarose, gélatine, alginate, dextrane, oxyde de fer, acier inoxydable, or, cuivre, chlorure d'argent, polycarbonate, polydiméthylsiloxane, polyéthylène, acrylonitrile butadiène styrène, polymères de cyclo-oléfine, copolymères de cyclo-oléfine, streptavidine, résine et un matériau biologique.

10. Procédé selon la revendication 8, dans lequel le substrat est un hydrogel compressible.

11. Procédé selon la revendication 9, dans lequel le matériau biologique est choisi dans le groupe constitué de : un tissu, une cellule, un organoïde, un tissu reconstitué ; et une matrice extracellulaire.

12. Procédé selon l'une quelconque des revendications 1 à 11, dans lequel le concaténeur de codes-barres code des informations spéciales et/ou des informations spatiales, éventuellement, dans lequel les informations spéciales sont sélectionnées dans le groupe constitué de : texte, images, coordonnées, graphiques, films, données de séquençage, QR codes, codes binaires et dossiers de santé.

13. Procédé selon l'une quelconque des revendications 1 à 12, dans lequel chacun des acides nucléiques est lié de manière covalente à au moins un autre des acides nucléiques par l'intermédiaire d'un seul élément photoréactif d'un domaine d'hybridation.

14. Procédé selon l'une quelconque des revendications 1 à 13, dans lequel chaque code-barres a une longueur d'au moins 5 nucléotides, éventuellement de 5 à 10 nucléotides.

15. Procédé selon l'une quelconque des revendications 1 à 14, dans lequel les codes-barres d'acide nucléique peuvent être lus à l'aide :
(a) de technologies de séquençage des acides nucléiques ; ou
(b) d'une lecture par fluorescence ou colorimétrique.
